# NEUE EUROPÄISCHE PATENTSCHRIFT

(11) **EP 2 721 149 B2**
(45) Veröffentlichungstag und Bekanntmachung des Hinweises auf die Entscheidung über den Einspruch: **08.04.2026**
(45) Hinweis auf die Patenterteilung: 10.10.2018
(21) Anmeldenummer: 12728065.9
(22) Anmeldetag: 14.06.2012
(51) Int. Cl.: C12N 15/86

(54) **GESCHIRRSPÜLMITTEL MIT BLEICHKATALYSATOR UND PROTEASE**
DISHWASHING DETERGENT WITH BLEACHING CATALYST AND PROTEASE
DÉTERGENT POUR LAVE-VAISSELLE AVEC CATALYSEUR DE BLANCHIMENT ET PROTÉASE

(30) Priorität: 16.06.2011 DE 102011118037
(43) Veröffentlichungstag der Anmeldung: 23.04.2014
(73) Patentinhaber: Henkel AG & Co. KGaA, 40589 Düsseldorf (DE)
(72) Erfinder: MUßMANN, Nina, 47877 Willich (DE); EITING, Thomas, 40589 Düsseldorf (DE); BASTIGKEIT, Thorsten, 42279 Wuppertal (DE); BENDA, Konstantin, 40217 Düsseldorf (DE); HELLMUTH, Hendrik, 40237 Düsseldorf (DE); WEBER, Thomas, 41541 Dormagen (DE)
(86) Internationale Anmeldenummer: PCT/EP2012/061244
(87) Internationale Veröffentlichungsnummer: WO 2012/171980

(56) Entgegenhaltungen:
- WO-A1-2007/006305
- WO-A1-2008/064935
- WO-A1-2011/036263
- WO-A1-2011/036264
- WO-A1-97/22681
- WO-A2-2009/021867
- DE-A1- 102006 022 216
- DE-A1- 102007 059 968

## Beschreibung

Die Erfindung liegt auf dem Gebiet der Geschirrspülmittel. Die Erfindung betrifft insbesondere bleichehaltige Geschirrspülmittel, die Proteasen enthalten, sowie Verfahren, in denen derartige Mittel angewendet werden. Die Erfindung betrifft ferner Verwendungen derartiger Mittel.

Geschirrspülmittel stehen dem Verbraucher in einer Vielzahl von Angebotsformen zur Verfügung. Neben den traditionellen flüssigen Handgeschirrspülmitteln haben mit der Verbreitung von Haushaltsgeschirrspülmaschinen insbesondere die maschinellen Geschirrspülmittel eine große Bedeutung erlangt. Diese maschinellen Geschirrspülmittel werden dem Verbraucher typischerweise in fester Form, beispielsweise als Pulver oder als Tabletten, angeboten.

Eines der Hauptziele der Hersteller maschineller Reinigungsmittel ist die Verbesserung der Reinigungsleistung dieser Mittel, wobei in jüngster Zeit ein verstärktes Augenmerk auf die Reinigungsleistung bei Niedrigtemperatur-Reinigungsgängen bzw. in Reinigungsgängen mit verringertem Wasserverbrauch gelegt wird.

Bleichbare Anschmutzungen, insbesondere Teeanschmutzungen, stellen jedoch hartnäckige Anschmutzungen dar, die oftmals nicht zufrieden stellend entfernt werden. Moderne Geschirrspülmittel, insbesondere maschinelle Geschirrspülmittel, genügen im Hinblick auf die Beseitigung von derartigen Anschmutzungen oft nicht den gestellten Anforderungen. Es besteht daher weiterhin Bedarf an Geschirrspülmitteln und hierunter insbesondere an maschinellen Geschirrspülmitteln, die bleichbare Anschmutzungen zuverlässig entfernen, insbesondere auch bei tieferen Reinigungstemperaturen.

Diesbezüglich ist in der europäischen Patentanmeldung EP 846155 offenbart, dass durch den Zusatz von Lipase die Wirkung von Bleichmitteln auf Teeanschmutzungen erhöht wird. Aus der europäischen Patentanmeldung EP 476257 geht die Verwendung von Aminosäuren als Bleichstabilisatoren hervor. Aus WO 2011/036263 und WO 2011/036264 sind verschiedene Bleichsysteme für Reinigungsmittel und Mangan als Enzymstabilisator bekannt. Aus WO 97/22681 sind Geschirrspülmittelzusammensetzungen mit Cobalt-Bleichkatalysator bekannt.

Eine verbesserte Bleichleistung durch Kombinationen von definierten Proteasen und Mangan-Bleichkatalysatoren geht aus dem Stand der Technik nicht hervor.

Der vorliegenden Erfindung liegt die Aufgabe zugrunde, Geschirrspülmittel mit einer verbesserten Reinigungsleistung an bleichbaren Anschmutzungen, insbesondere Teeanschmutzungen, bereitzustellen.

Gegenstand der Erfindung ist ein Geschirrspülmittel gemäß Anspruch 1, ein Verfahren gemäß Anspruch 7 zur Entfernung von Anschmutzungen, insbesondere Teeanschmutzungen, auf harten Oberflächen, insbesondere Geschirr, sowie eine Verwendung des erfindungsgemäßen Geschirrspülmittels zur Entfernung von Anschmutzungen, insbesondere Teeanschmutzungen, auf harten Oberflächen, insbesondere Geschirr, gemäß Anspruch 8.

Anspruch 1 betrifft ein Geschirrspülmittel, umfassend eine Wasserstoffperoxidquelle, einen Bleichkatalysator und eine Protease, dadurch gekennzeichnet, dass der Bleichkatalysator ausgewählt ist aus der Gruppe der Komplexe des Mangans mit 1,4,7-trimethyl-1,4,7-triazacyclononan (Me-TACN) oder 1,2,4,7-tetramethyl-1,4,7-triazacyclononan (Me/Me-TACN), die Protease eine Aminosäuresequenz umfasst, die zu der in SEQ ID NO. 3 angegebenen Aminosäuresequenz identisch ist und die Wasserstoffperoxidquelle Natriumpercarbonat ist.

Überraschenderweise wurde festgestellt, dass derartige Geschirrspülmittel, die solche Proteasen in Kombination mit solch einem Bleichkatalysator umfassen, eine sehr gute Bleichleistung aufweisen und folglich eine sehr gute Reinigungsleistung an bleichbaren Anschmutzungen, insbesondere Teeanschmutzungen, zeigen. Im erfindungsgemäßen Mittel liegt folglich ein Synergismus zwischen der entsprechenden Protease und dem Bleichkatalysator vor hinsichtlich der Reinigungsleistung, insbesondere hinsichtlich der Reinigungsleistung an Teeanschmutzungen. Weitere bevorzugte Ausgestaltungen erfindungsgemäßer Mittel zeigen solche vorteilhaften Reinigungsleistungen auch bei niedrigen Temperaturen und/oder bei kurzen Spülgängen. Eine niedrige Temperatur im Sinne der Erfindung liegt vorzugsweise zwischen 10°C und 50°C, vorzugsweise zwischen 15°C und 45°C und besonders bevorzugt zwischen 20°C und 40°C vor. Ein kurzer Spülgang dauert vorzugsweise maximal 60 Minuten, 45 Minuten oder lediglich maximal 30 Minuten. Weitere bevorzugte Ausgestaltungen erfindungsgemäßer Mittel zeigen ferner eine verbesserte Klarspülleistung.

Die Reinigungsleistung beschreibt das Vermögen eines Geschirrspülmittels, insbesondere eines maschinellen Geschirrspülmittels, eine vorhandene Anschmutzung teilweise oder vollständig zu entfernen. Im Rahmen der Erfindung weist sowohl das Geschirrspülmittel, welches die Protease umfasst bzw. die durch dieses Mittel gebildete Reinigungsflotte, als auch die Protease selbst eine jeweilige Reinigungsleistung auf. Die Reinigungsleistung des Enzyms trägt somit zur Reinigungsleistung des Mittels bzw. der durch das Mittel gebildeten Reinigungsflotte bei.

Unter Reinigungsflotte wird diejenige das Geschirrspülmittel enthaltende Gebrauchslösung verstanden, die auf die harten Oberflächen einwirkt und damit mit den auf den harten Oberflächen vorhandenen Anschmutzungen in Kontakt kommt. Üblicherweise entsteht die Reinigungsflotte, wenn der Reinigungsvorgang beginnt und das Geschirrspülmittel beispielsweise in einer Spülmaschine oder in einem anderen geeigneten Behältnis mit Wasser verdünnt wird.

Überraschenderweise sind es genau solche Proteasen, die im Zusammenwirken mit dem Bleichkatalysator in einem erfindungsgemäßen Mittel eine verbesserte Reinigungsleistung an bleichbaren Anschmutzungen, insbesondere Teeanschmutzungen, bewirken. Vorzugsweise liegt diesbezüglich ein synergistisches Zusammenwirken vor. SEQ ID NO. 1 ist die Aminosäuresequenz von Subtilisin 309. Das vorteilhafte Zusammenwirken mit dem Bleichkatalysator tritt bei solchen Proteasen auf, die eine Aminosäuresequenz umfassen, die zu der in SEQ ID NO. 3 angegebenen Aminosäuresequenz identisch ist.

Ein erfindungsgemäßes Geschirrspülmittel enthält die Protease zunehmend bevorzugt in einer Menge von 1 x 10⁻⁸-10 Gew.-%, von 0,00001-2 Gew.-%, von 0,001-1 Gew.-%, von 0,007 bis 0,8 Gew.-%, von 0,025 bis 0,5 Gew.-% und besonders bevorzugt von 0,04 bis 0,38 Gew.-%, bezogen auf den Gesamtproteingehalt der Protease.

Die Proteinkonzentration kann mit Hilfe bekannter Methoden, zum Beispiel dem BCA-Verfahren (Bicinchoninsäure; 2,2'-Bichinolyl-4,4'-dicarbonsäure) oder dem Biuret-Verfahren (A. G. Gornall, C. S. Bardawill und M.M. David, J. Biol. Chem., 177 (1948), S. 751-766) bestimmt werden.

Die Protease kann ferner an Trägerstoffen adsorbiert und/oder in Hüllsubstanzen eingebettet sein, um sie gegen vorzeitige Inaktivierung zu schützen. In der Reinigungsflotte, also unter Anwendungsbedingungen, wird das Enzym dann freigesetzt und kann seine katalytische Wirkung entfalten.

Ein erfindungsgemäßes Geschirrspülmittel umfasst ferner einen Bleichaktivator. Bei diesen Stoffen handelt es sich vorzugsweise um bleichverstärkende Übergangsmetallsalze bzw. Übergangsmetallkomplexe wie beispielsweise Mn-, Fe-, Co-, Ru - oder Mo-Salenkomplexe oder -carbonylkomplexe. Auch Mn-, Fe-, Co-, Ru-, Mo-, Ti-, V- und Cu-Komplexe mit N-haltigen Tripod-Liganden sowie Co-, Fe-, Cu- und Ru-Amminkomplexe sind als Bleichkatalysatoren verwendbar.

Mit besonderem Vorzug werden Komplexe des Mangans in der Oxidationsstufe II, III, IV oder IV eingesetzt, die vorzugsweise einen oder mehrere makrocyclische(n) Ligand(en) mit den Donorfunktionen N, NR, PR, O und/oder S enthalten. Vorzugsweise werden Liganden eingesetzt, die Stickstoff-Donorfunktionen aufweisen. Dabei ist es besonders bevorzugt, Bleichkatalysator(en) in den erfindungsgemäßen Mitteln einzusetzen, welche als makromolekularen Liganden 1,4,7-Trimethyl-1,4,7-triazacyclononan (Me-TACN), 1,4,7-Triazacyclononan (TACN), 1,5,9-Trimethyl-1,5,9-triazacyclododecan (Me-TACD), 2-Methyl-1,4,7-trimethyl-1,4,7-triazacyclononan (Me/Me-TACN) und/oder 2-Methyl-1,4,7-triazacyclononan (Me/TACN) enthalten. Geeignete Mangankomplexe sind beispielsweise [Mn^{III}₂(µ-O)₁(µ-OAc)₂(TACN)₂](ClO₄)₂, [Mn^{III}Mn^{IV}(µ-O)₂(µ-OAc)₁(TACN)₂](BPh₄)₂, [Mn^{IV}4(µ-O)₆(TACN)₄](ClO₄)₄, [Mn^{III}₂(µ-O)₁(µ-OAc)₂(Me-TACN)₂](ClO₄)₂, [Mn^{III}Mn^{IV}(µ-O)₁(µ-OAc)₂(Me-TACN)₂](ClO₄)₃, [Mn^{IV}₂(µ-O)₃(Me-TACN)₂](PF₆)₂ und [Mn^{IV}₂(µ-O)₃(Me/Me-TACN)₂](PF₆)₂(OAc = OC(O)CH₃).

Geschirrspülmittel, insbesondere maschinelle Geschirrspülmittel, dadurch gekennzeichnet, dass sie einen Bleichkatalysator ausgewählt aus der Gruppe der bleichverstärkenden Übergangsmetallsalze und Übergangsmetallkomplexe, vorzugsweise aus der Gruppe der Komplexe des Mangans mit 1,4,7-trimethyl-1,4,7-triazacyclononan (Me-TACN) oder 1,2,4,7-tetramethyl-1,4,7-triazacyclononan (Me/Me-TACN) enthalten, werden erfindungsgemäß bevorzugt, da durch die vorgenannten Bleichkatalysatoren insbesondere das Reinigungsergebnis signifikant verbessert werden kann.

Die vorgenannten bleichverstärkenden Übergangsmetallkomplexe, insbesondere mit den Zentralatomen Mn und Co werden vorzugsweise in einer Menge bis zu 5 Gew.-%, insbesondere von 0,0025 Gew.-% bis 1 Gew.-% und besonders bevorzugt von 0,01 Gew.-% bis 0,30 Gew.-%, jeweils bezogen auf das Gesamtgewicht der bleichkatalysatorhaltigen Mittel, eingesetzt. In speziellen Fällen kann jedoch auch mehr Bleichkatalysator eingesetzt werden.

Ein erfindungsgemäßes Geschirrspülmittel umfasst ferner eine Wasserstoffperoxidquelle. Hierbei handelt es sich um Verbindungen, die in Wasser H₂O₂ liefern bzw. liefern können. Bei der Wasserstoffperoxidquelle handelt es sich vorzugsweise um ein Bleichmittel, wobei Sauerstoffbleichmittel erfindungsgemäß bevorzugt werden.

Unter den als Bleichmittel dienenden, in Wasser H₂O₂ liefernden Verbindungen haben das Natriumpercarbonat, das Natriumperborattetrahydrat und das Natriumperboratmonohydrat besondere Bedeutung. Weitere brauchbare Bleichmittel sind beispielsweise Peroxypyrophosphate, Citratperhydrate sowie H₂O₂ liefernde persaure Salze oder Persäuren, wie Perbenzoate, Peroxophthalate, Diperazelainsäure, Phthaloiminopersäure oder Diperdodecandisäure.

Weiterhin können auch Bleichmittel aus der Gruppe der organischen Bleichmittel eingesetzt werden. Typische organische Bleichmittel sind die Diacylperoxide, wie z.B. Dibenzoylperoxid. Weitere typische organische Bleichmittel sind die Peroxysäuren, wobei als Beispiele besonders die Alkylperoxysäuren und die Arylperoxysäuren genannt werden.

Vorzugsweise ist die Wasserstoffperoxidquelle in einer Menge von 2-30 Gew.-% und zunehmend bevorzugt von 4-25 Gew.-%, von 5-20 Gew.-% und besonders bevorzugt von 6-15 Gew.-% in dem erfindungsgemäßen Geschirrspülmittel enthalten, jeweils bezogen auf das Gesamtgewicht des Geschirrspülmittels. Bevorzugte Geschirrspülmittel sind ferner dadurch gekennzeichnet, dass das Geschirrspülmittel, jeweils bezogen auf das Gesamtgewicht des Geschirrspülmittels, 2 bis 20 Gew.-%, vorzugsweise 3 bis 18 Gew.-% und insbesondere 4 bis 15 Gew.-% Natriumpercarbonat enthält.

Besonders bevorzugte Ausführungsformen erfindungsgemäßer Geschirrspülmittel sind folglich dadurch gekennzeichnet, dass der Bleichkatalysator ausgewählt ist aus der Gruppe der bleichverstärkenden Übergangsmetallsalze und Übergangsmetallkomplexe, vorzugsweise aus der Gruppe der Komplexe des Mangans mit 1,4,7-trimethyl-1,4,7-triazacyclononan (Me-TACN) oder 1,2, 4,7-tetramethyl-1,4,7-triazacyclononan (Me/Me-TACN), und/oder die Wasserstoffperoxidquelle Natriumpercarbonat, Natriumperborattetrahydrat oder Natriumperboratmonohydrat oder eine Kombination hiervon ist. Ganz besonders bevorzugt ist der Bleichkatalysator ein Komplex des Mangans mit 1,4,7-trimethyl-1,4,7-triazacyclononan (Me-TACN), insbesondere [Mn^{IV}₂(µ-O)₃(Me-TACN)₂](PF₆)₂, oder 1,2, 4,7-tetramethyl-1,4,7-triazacyclononan (Me/Me-TACN) oder eine Mischung hieraus, und die Wasserstoffperoxidquelle Natriumpercarbonat. In den genannten Kombinationen liegen der Bleichkatalysator und die Wasserstoffperoxidquelle vorzugsweise in den vorstehend jeweils genannten Mengen vor.

Erfindungsgemäße Geschirrspülmittel, insbesondere maschinelle Geschirrspülmittel, können ferner Bleichaktivatoren enthalten, beispielsweise um beim Reinigen bei Temperaturen von 60°C und darunter eine verbesserte Bleichwirkung zu erreichen. Als Bleichaktivatoren können Verbindungen, die unter Perhydrolysebedingungen aliphatische Peroxocarbonsäuren mit vorzugsweise 1 bis 10 C-Atomen, insbesondere 2 bis 4 C-Atomen, und/oder gegebenenfalls substituierte Perbenzoesäure ergeben, eingesetzt werden. Geeignet sind Substanzen, die O- und/oder N-Acylgruppen der genannten C-Atomzahl und/oder gegebenenfalls substituierte Benzoylgruppen tragen. Bevorzugt werden mehrfach acylierte Alkylendiamine, wobei sich Tetraacetylethylendiamin (TAED) als besonders geeignet erwiesen hat.

Diese Bleichaktivatoren, insbesondere TAED, werden vorzugsweise in Mengen von 0,1-10 Gew.-%, insbesondere 0,1-8 Gew.-%, besonders 2-8 Gew.-% und besonders bevorzugt 2-6 Gew.-%, jeweils bezogen auf das Gesamtgewicht der bleichaktivatorhaltigen Mittel, eingesetzt.

Die Protease umfasst eine Aminosäuresequenz, die zu der in SEQ ID NO. 3 angegebenen Aminosäuresequenz zu 100% identisch ist.

Die Bestimmung der Identität von Nukleinsäure- oder Aminosäuresequenzen erfolgt durch einen Sequenzvergleich. Solch ein Vergleich erfolgt dadurch, dass ähnliche Abfolgen in den Nukleotidsequenzen oder Aminosäuresequenzen einander zugeordnet werden. Dieser Sequenzvergleich erfolgt vorzugsweise basierend auf dem im Stand der Technik etablierten und üblicherweise genutzten BLAST-Algorithmus (vgl. beispielsweise Altschul, S.F., Gish, W., Miller, W., Myers, E.W. & Lipman, D.J. (1990) "Basic local alignment search tool." J. Mol. Biol. 215:403-410, und Altschul, Stephan F., Thomas L. Madden, Alejandro A. Schaffer, Jinghui Zhang, Hheng Zhang, Webb Miller, and David J. Lipman (1997): "Gapped BLAST and PSI-BLAST: a new generation of protein database search programs"; Nucleic Acids Res., 25, S.3389-3402) und geschieht prinzipiell dadurch, dass ähnliche Abfolgen von Nukleotiden oder Aminosäuren in den Nukleinsäure- bzw. Aminosäuresequenzen einander zugeordnet werden. Eine tabellarische Zuordnung der betreffenden Positionen wird als Alignment bezeichnet. Ein weiterer im Stand der Technik verfügbarer Algorithmus ist der FASTA-Algorithmus. Sequenzvergleiche (Alignments), insbesondere multiple Sequenzvergleiche, werden üblicherweise mit Computerprogrammen erstellt. Häufig genutzt werden beispielsweise die Clustal-Serie (vgl. beispielsweise Chenna et al. (2003): Multiple sequence alignment with the Clustal series of programs. Nucleic Acid Research 31, 3497-3500), T-Coffee (vgl. beispielsweise Notredame et al. (2000): T-Coffee: A novel method for multiple sequence alignments. J. Mol. Biol. 302, 205-217) oder Programme, die auf diesen Programmen bzw. Algorithmen basieren. Im Rahmen der vorliegenden Erfindung werden Sequenzvergleiche und Alignments bevorzugt mit dem Softwarepaket Vector NTI^{®} Advance 10.3.0 (Invitrogen Corporation, 1600 Faraday Avenue, Carlsbad, Kalifornien, USA) mit den vorgegebenen Standard (Default)-Parametern erstellt.

Solch ein Vergleich erlaubt eine Aussage über die Ähnlichkeit der verglichenen Sequenzen zueinander. Sie wird üblicherweise in Prozent Identität, das heißt dem Anteil der identischen Nukleotide oder Aminosäurereste an denselben bzw. in einem Alignment einander entsprechenden Positionen, angegeben. Der weiter gefasste Begriff der Homologie bezieht bei Aminosäuresequenzen konservierte Aminosäure-Austausche in die Betrachtung mit ein, also Aminosäuren mit ähnlichen Eigenschaften, da diese innerhalb des Proteins meist ähnliche Aktivitäten bzw. Funktionen ausüben. Daher kann die Ähnlichkeit der verglichenen Sequenzen auch Prozent Homologie oder Prozent Ähnlichkeit angegeben sein. Identitäts- und/oder Homologieangaben können über ganze Polypeptide oder Gene oder nur über einzelne Bereiche getroffen werden. Homologe bzw. identische Bereiche von verschiedenen Nukleinsäure- oder Aminosäuresequenzen sind daher durch Übereinstimmungen in den Sequenzen definiert. Sie weisen oftmals gleiche oder ähnliche Funktionen auf. Sie können klein sein und nur wenige Nukleotide bzw. Aminosäuren umfassen. Oftmals üben solche kleinen Bereiche für die Gesamtaktivität des Proteins essentielle Funktionen aus. Es kann daher sinnvoll sein, Sequenzübereinstimmungen nur auf einzelne, gegebenenfalls kleine Bereiche zu beziehen. Soweit nicht anders angegeben beziehen sich Identitäts- bzw. Homologieangaben in der vorliegenden Anmeldung aber auf die Gesamtlänge der jeweils angegebenen Nukleinsäure- oder Aminosäuresäuresequenz.

In einer bevorzugten Ausführungsform der Erfindung handelt es sich bei einem erfindungsgemäßen Geschirrspülmittel um ein maschinelles Geschirrspülmittel. Als maschinelle Geschirrspülmittel werden nach Maßgabe dieser Anmeldung Zusammensetzungen bezeichnet, die zur Reinigung verschmutzten Geschirrs in einem maschinellen Geschirrspülverfahren eingesetzt werden können. Damit unterscheiden sich die erfindungsgemäßen maschinellen Geschirrspülmittel beispielsweise von den maschinellen Klarspülmitteln, die stets in Kombination mit maschinellen Geschirrspülmitteln eingesetzt werden und keine eigene Reinigungswirkung entfalten.

An maschinell gespültes Geschirr werden häufig höhere Anforderungen gestellt als an manuell gespültes Geschirr. So soll das Geschirr nach der maschinellen Reinigung nicht nur frei von Speiseresten sein, sondern beispielsweise auch keine weißlichen, auf Wasserhärte oder anderen mineralischen Salzen beruhenden Flecken aufweisen, die mangels Netzmittel aus eingetrockneten Wassertropfen stammen. Moderne maschinelle Geschirrspülmittel erfüllen diese Anforderungen durch die Integration reinigender und/oder pflegender und/oder wasserenthärtender und/oder klarspülaktiver Wirkstoffe und sind dem Verbraucher beispielsweise als "2in1"- oder "3in1" Geschirrspülmittel bekannt. Als für den Reinigungs- wie für den Klarspülerfolg wesentlichen Bestandteil enthalten die maschinellen Geschirrspülmittel Gerüststoffe. Diese Gerüststoffe erhöhen zum einen die Alkalität der Reinigungsflotte, wobei mit steigender Alkalität Fette und Öle emulgiert und verseift werden, und vermindern zum anderen durch Komplexierung der in der wässrigen Flotte enthaltenen Calciumionen die Wasserhärte der Reinigungsflotte.

In einer weiteren Ausführungsform der Erfindung ist das Geschirrspülmittel dadurch gekennzeichnet, dass es in fester Form vorliegt. Hierunter wird jede feste Konfektionierungsform verstanden, beispielsweise Pulver, Granulate oder Extrudate.

Ein erfindungsgemäßes pulverförmiges Mittel kann beispielsweise als rieselfähiges Pulver vorliegen, insbesondere mit einem Schüttgewicht von 300 g/l bis 1200 g/l, insbesondere 500 g/l bis 900 g/l oder 600 g/l bis 850 g/l.

Die erfindungsgemäßen Geschirrspülmittel, insbesondere die maschinellen Geschirrspülmittel, liegen vorzugsweise in Form eines Formkörpers, insbesondere eines Kompaktats, vor allem einer Tablette, vor. Bei dem Formkörper kann es sich aber beispielsweise auch um ein Granulat handeln, das in einem Beutel oder einer Gießform enthalten ist.

Erfindungsgemäße Mittel können als einphasige oder mehrphasige Produkte konfektioniert werden. Bevorzugt sind insbesondere maschinelle Geschirrspülmittel mit einer, zwei, drei oder vier Phasen. Maschinelle Geschirrspülmittel, dadurch gekennzeichnet, dass sie in Form einer vorgefertigten Dosiereinheit mit zwei oder mehr Phasen vorliegen, sind besonders bevorzugt. Besonders bevorzugt sind insbesondere zwei- oder mehrphasige Tabletten, beispielsweise Zweischichttabletten, insbesondere Zweischichttabletten mit Mulde und einem in der Mulde befindlichen Formkörper.

Erfindungsgemäße maschinelle Geschirrspülmittel werden vorzugsweise zu Dosiereinheiten vorkonfektioniert. Diese Dosiereinheiten umfassen vorzugsweise die für einen Reinigungsgang notwendige Menge an wasch- oder reinigungsaktiven Substanzen. Bevorzugte Dosiereinheiten weisen ein Gewicht zwischen 12 und 30 g, bevorzugt zwischen 14 und 26 g und insbesondere zwischen 15 und 22 g auf.

Das Volumen der vorgenannten Dosiereinheiten sowie deren Raumform sind mit besonderem Vorzug so gewählt, dass eine Dosierbarkeit der vorkonfektionierten Einheiten über die Dosierkammer einer Geschirrspülmaschine gewährleistet ist. Das Volumen der Dosiereinheit beträgt daher bevorzugt zwischen 10 und 35 ml, vorzugsweise zwischen 12 und 30 ml und insbesondere zwischen 15 und 25 ml.

Die erfindungsgemäßen maschinellen Geschirrspülmittel, insbesondere die vorgefertigten Dosiereinheiten, weisen in einer bevorzugten Ausführungsform eine wasserlösliche Umhüllung auf.

Die Herstellung erfindungsgemäßer fester Mittel bietet keine Schwierigkeiten und kann auf bekannte Weise, zum Beispiel durch Sprühtrocknen oder Granulation, erfolgen, wobei Enzyme und eventuelle weitere thermisch empfindliche Inhaltsstoffe wie zum Beispiel Bleichmittel gegebenenfalls später separat zugesetzt werden. Zur Herstellung erfindungsgemäßer Mittel mit erhöhtem Schüttgewicht, insbesondere im Bereich von 650 g/l bis 950 g/l, ist ein einen Extrusionschritt aufweisendes Verfahren bevorzugt.

Die Herstellung erfindungsgemäßer Formkörper, insbesondere der Reinigungsmitteltabletten, erfolgt bevorzugt in dem Fachmann bekannter Weise durch Verpressung partikulärer Ausgangssubstanzen. Zur Herstellung der Tabletten wird das Vorgemisch in einer so genannten Matrize zwischen zwei Stempeln zu einem festen Komprimat verdichtet. Dieser Vorgang, der im Folgenden kurz als Tablettierung bezeichnet wird, gliedert sich in vier Abschnitte: Dosierung, Verdichtung (elastische Verformung), plastische Verformung und Ausstoßen. Die Tablettierung erfolgt dabei vorzugsweise auf so genannten Rundläuferpressen.

Bei der Tablettierung mit Rundläuferpressen hat es sich als vorteilhaft erwiesen, die Tablettierung mit möglichst geringen Gewichtschwankungen der Tablette durchzuführen. Auf diese Weise lassen sich auch die Härteschwankungen der Tablette reduzieren. Die Minimierung von Gewichtschwankungen kann auf folgende Weise erzielt werden:
- Verwendung von Kunststoffeinlagen mit geringen Dickentoleranzen
- Geringe Umdrehungszahl des Rotors
- Große Füllschuhe
- Abstimmung des Füllschuhflügeldrehzahl auf die Drehzahl des Rotors
- Füllschuh mit konstanter Pulverhöhe
- Entkopplung von Füllschuh und Pulvervorlage

Die zur Tablettierung vorgesehen Inhaltsstoffe können in Form eines gemeinsamen teilchenförmigen Vorgemisches zeitgleich oder in Form einzelner, separater Pulver oder Granulate zeitlich versetzt oder zeitgleich in die Matrize eingefüllt werden, wobei die Dosierung eines vorgefertigten teilchenförmigen Vorgemisches bevorzugt ist.

Es wurde überraschenderweise festgestellt, dass sich zur Herstellung der Formkörper eingesetzte Granulate besonders gut verpressen lassen. So können vorzugsweise unter Anwendung einer Presskraft von 40 bis 65 kN, besonders bevorzugt von 48 bis 60 kN, Kompaktate mit einer Härte im Bereich von 150 bis 250 N, insbesondere im Bereich von 200 bis 230 N erhalten werden, die darüber hinaus ein besonders gutes Rieselverhalten aufweisen. Die Granulate lassen sich somit vorzugsweise mit relativ geringer Presskraft zu Kompaktaten mit relativ hoher Härte verpressen, die darüber hinaus vorzugsweise ein sehr gutes Rieselverhalten aufweisen. Entsprechend ist es umgekehrt von Vorteil, dass zur Herstellung von Kompaktaten geringerer Härte vorzugsweise eine geringere Presskraft aufgewandt werden muss, als zur Herstellung üblicher Kompaktate.

Erfindungsgemäße Zusammensetzungen, insbesondere Formkörper, enthalten in einer weiteren bevorzugten Ausführungsform Polyvinylpyrrolidon-Partikel. Diese Partikel erleichtern unter anderem den Zerfall der Formkörper und dienen insofern als Desintegrationshilfsmittel bzw. Tablettensprengmittel. Es hat sich erfindungsgemäß als besonders vorteilhaft herausgestellt, Polyvinylpyrrolidon-Partikel mit einem mittleren Teilchendurchmesser von 100 bis 150 µm, insbesondere mit einem mittleren Teilchendurchmesser von 110 bis 130 µm, einzusetzen.

Unter dem Begriff "mittlerer Teilchendurchmesser" bzw. "mittlerer Durchmesser" ist im Rahmen der vorliegenden Erfindung der volumenmittlere D₅₀ Teilchendurchmesser zu verstehen, der nach üblichen Verfahren bestimmt werden kann. Der volumenmittlere D₅₀ Teilchendurchmesser ist derjenige Punkt in der Teilchengrößenverteilung, bei dem 50 Vol.-% der Teilchen einen kleineren und 50 Vol.-% der Teilchen einen größeren Durchmesser aufweisen. Die mittleren Teilchendurchmesser können insbesondere mit Hilfe dynamischer Lichtstreuung bestimmt werden, die üblicherweise an verdünnten Suspensionen, die z.B. 0,01 bis 1 Gew.-% Partikel enthalten, durchgeführt wird.

Besonders bevorzugt weisen die PVP-Partikel nicht nur einen mittleren Teilchendurchmesser von 100 bis 150 µm, insbesondere von 110 bis 130 µm, auf, sondern darüber hinaus liegt die Teilchengröße der eingesetzten Partikel vorzugsweise vollständig in den angegebenen Intervallen. Dies wird dadurch sichergestellt, dass Korngrößenfraktionen mit den angegebenen Teilchengrößen, die durch ein Siebverfahren erhalten wurden, eingesetzt werden.

Die PVP-Partikel sind in erfindungsgemäßen Zusammensetzungen, insbesondere Formkörpern, vorzugsweise in einer Menge von 0,1 bis 5 Gew.-%, insbesondere in einer Menge von 0,2 bis 3 Gew.-%, vor allem in einer Menge von 0,3 bis 1,8 Gew.-%, enthalten.

Die Wirkung von Sprengmitteln besteht in der Regel darin, dass sie bei Wasserzutritt ihr Volumen vergrößern, wobei sich einerseits das Eigenvolumen vergrößert (Quellung), andererseits aber auch über die Freisetzung von Gasen ein Druck erzeugt werden kann, der die Tablette in kleinere Partikel zerfallen lässt. Zusätzlich oder alternativ zu den PVP-Partikeln können auch weitere Sprengmittel in erfindungsgemäßen Zusammensetzungen, insbesondere Formkörpern, enthalten sein, beispielsweise Carbonat/Citronensäure-Systeme oder Carbonat in Kombination mit anderen organischen Säuren, synthetische Polymere oder natürliche Polymere bzw. modifizierte Naturstoffe wie Cellulose und Stärke und ihre Derivate sowie Alginate oder Casein-Derivate. Weiterhin können als weitere Sprengmittel auch gasentwickelnde Brausesysteme eingesetzt werden. Bevorzugte Brausesysteme bestehen aus mindestens zwei Bestandteilen, die miteinander unter Gasbildung reagieren, beispielsweise aus Alkalimetallcarbonat und/oder -hydrogencarbonat sowie einem Acidifizierungsmittel, das geeignet ist, aus den Alkalimetallsalzen in wässriger Lösung Kohlendioxid freizusetzen. Ein Acidifizierungsmittel, das aus den Alkalisalzen in wässriger Lösung Kohlendioxid freisetzt, ist beispielsweise die Citronensäure.

Die weiteren Desintegrationshilfsmittel werden, sofern verwendet, vorzugsweise in Mengen von 0,1 bis 10 Gew.-%, vorzugsweise 0,2 bis 5 Gew.-% und insbesondere 0,5 bis 2 Gew.-%, jeweils bezogen auf das Gesamtgewicht des desintegrationshilfsmittelhaltigen Mittels, eingesetzt.

In einer weiteren Ausführungsform der Erfindung ist das Geschirrspülmittel dadurch gekennzeichnet, dass es in flüssiger, gelförmiger oder pastöser Form vorliegt.

Als flüssige Mittel können hierbei alle flüssigen bzw. fließfähigen Darreichungsformen dienen. "Fließfähig" im Sinne der vorliegenden Anmeldung sind dabei Mittel, welche gießbar sind und Viskositäten bis hin zu mehreren 10.000 mPas aufweisen können. Die Viskosität kann mit üblichen Standardmethoden (beispielsweise Brookfield-Viskosimeter LVT-II bei 20 U/min und 20°C, Spindel 3) gemessen werden und liegt vorzugsweise im Bereich von 5 bis 10000 mPas. Bevorzugte Mittel haben Viskositäten von 10 bis 8000 mPas, wobei Werte zwischen 120 bis 3000 mPas besonders bevorzugt sind. Ein flüssiges Mittel im Rahmen der vorliegenden Erfindung kann daher auch gelförmig oder pastenförmig sein, es kann als homogene Lösung oder Suspension vorliegen, sowie beispielsweise versprühbar oder in sonstigen üblichen Darreichungsformen konfektioniert sein.

Flüssige beziehungsweise pastöse erfindungsgemäße Mittel in Form von übliche Lösungsmittel enthaltenden Lösungen werden in der Regel durch einfaches Mischen der Inhaltsstoffe, die in Substanz oder als Lösung in einen automatischen Mischer gegeben werden können, hergestellt.

Ausführungsformen der vorliegenden Erfindung umfassen somit alle festen, pulverförmigen, flüssigen, gelförmigen oder pastösen Darreichungsformen der Mittel, die gegebenenfalls auch aus mehreren Phasen bestehen können sowie in komprimierter oder nicht komprimierter Form vorliegen können. Ein erfindungsgemäßes Mittel kann ferner in einem Behältnis, vorzugsweise einem luftdurchlässigen Behältnis, verpackt sein, aus dem es kurz vor Gebrauch oder während des Reinigungsvorgangs freigesetzt wird.

In einer weiteren Ausführungsform der Erfindung umfasst ein erfindungsgemäßes Geschirrspülmittel ferner mindestens einen weiteren Inhaltsstoff, der ausgewählt ist aus der Gruppe bestehend aus Gerüststoff, Tensid, anionisches Polymer sowie Kombinationen hiervon. In einer weiteren Ausführungsform der Erfindung ist ein erfindungsgemäßes Geschirrspülmittel phosphatfrei. Erfindungsgemäße phosphatfreie Geschirrspülmittel sind insbesondere unter Umweltaspekten vorteilhaft.

Vorzugsweise werden die Inhaltsstoffe der Mittel aufeinander abgestimmt. Bevorzugt sind Synergien hinsichtlich der Reinigungsleistung und/oder der Klarspülleistung und/oder der Belagsinhibierung. Besonders bevorzugt sind Synergien, die in einem Temperaturbereich zwischen 10°C und 60°C vorhanden sind, insbesondere in einem Temperaturbereich von 10°C bis 50°C, von 10°C bis 40°C, von 10°C bis 30°C, von 15°C bis 30°C von 10°C bis 25°C und von 15°C bis 25°C.

Zur Gruppe bevorzugter Gerüststoffe (Builder) zählen insbesondere die Citrate sowie die Carbonate und die organischen Cobuilder. Die Bezeichnung "Citrat" umfasst dabei ebenso die Citronensäure wie auch deren Salze, insbesondere deren Alkalimetallsalze. Besonders bevorzugte erfindungsgemäße Geschirrspülmittel, insbesondere maschinelle Geschirrspülmittel, enthalten Citronensäure und Citrat, vorzugsweise Natriumcitrat, in Mengen von 5 bis 60 Gew.-%, vorzugsweise 10 bis 50 Gew.-% und insbesondere 15 bis 40 Gew.-%.

Besonders bevorzugt ist der Einsatz von Carbonat(en) und/oder Hydrogencarbonat(en), vorzugsweise Alkalicarbonat(en), besonders bevorzugt Natriumcarbonat, in Mengen von 5 bis 50 Gew.-%, vorzugsweise von 10 bis 40 Gew.-% und insbesondere von 15 bis 30 Gew.-%, jeweils bezogen auf das Gewicht des Geschirrspülmittels.

Als organische Cobuilder sind insbesondere Polycarboxylate / Polycarbonsäuren und Phosphonate zu nennen. Diese Stoffklassen werden nachfolgend beschrieben.

Brauchbare organische Gerüstsubstanzen sind beispielsweise die in Form der freien Säure und/oder ihrer Natriumsalze einsetzbaren Polycarbonsäuren, wobei unter Polycarbonsäuren solche Carbonsäuren verstanden werden, die mehr als eine Säurefunktion tragen. Beispielsweise sind dies Adipinsäure, Bernsteinsäure, Glutarsäure, Äpfelsäure, Weinsäure, Maleinsäure, Fumarsäure, Zuckersäuren, Aminocarbonsäuren, Nitrilotriessigsäure (NTA) sowie Mischungen aus diesen. Die freien Säuren besitzen neben ihrer Builderwirkung typischerweise auch die Eigenschaft einer Säuerungskomponente und dienen somit auch zur Einstellung eines niedrigeren und milderen pH-Wertes von erfindungsgemäßen Mitteln. Insbesondere sind hierbei Bernsteinsäure, Glutarsäure, Adipinsäure, Gluconsäure und beliebige Mischungen aus diesen zu nennen.

Die komplexbildenden Phosphonate umfassen neben der 1-Hydroxyethan-1,1-diphosphonsäure eine Reihe unterschiedlicher Verbindungen wie beispielsweise Diethylentriaminpenta(methylenphosphonsäure) (DTPMP). In dieser Anmeldung bevorzugt sind insbesondere Hydroxyalkan- bzw. Aminoalkanphosphonate. Unter den Hydroxyalkanphosphonaten ist das 1-Hydroxyethan-1,1-diphosphonat (HEDP) von besonderer Bedeutung als Cobuilder. Es wird vorzugsweise als Natriumsalz eingesetzt, wobei das Dinatriumsalz neutral und das Tetranatriumsalz alkalisch (pH 9) reagiert. Als Aminoalkanphosphonate kommen vorzugsweise Ethylendiamintetramethylenphosphonat (EDTMP), Diethylentriaminpentamethylenphosphonat (DTPMP) sowie deren höhere Homologe in Frage. Sie werden vorzugsweise in Form der neutral reagierenden Natriumsalze, z. B. als Hexanatriumsalz der EDTMP bzw. als Hepta- und Octa-Natriumsalz der DTPMP, eingesetzt. Als Builder wird dabei aus der Klasse der Phosphonate bevorzugt HEDP verwendet. Die Aminoalkanphosphonate besitzen zudem ein ausgeprägtes Schwermetallbindevermögen. Dementsprechend kann es, insbesondere wenn die Mittel auch Bleiche enthalten, bevorzugt sein, Aminoalkanphosphonate, insbesondere DTPMP, einzusetzen, oder Mischungen aus den genannten Phosphonaten zu verwenden.

Ein im Rahmen dieser Anmeldung bevorzugtes Geschirrspülmittel, insbesondere maschinelles Geschirrspülmittel, enthält ein oder mehrere Phosphonat(e) aus der Gruppe
a) Aminotrimethylenphosphonsäure (ATMP) und/oder deren Salze;
b) Ethylendiamintetra(methylenphosphonsäure) (EDTMP) und/oder deren Salze;
c) Diethylentriaminpenta(methylenphosphonsäure) (DTPMP) und/oder deren Salze;
d) 1-Hydroxyethan-1,1-diphosphonsäure (HEDP) und/oder deren Salze;
e) 2-Phosphonobutan-1,2,4-tricarbonsäure (PBTC) und/oder deren Salze;
f) Hexamethylendiamintetra(methylenphosphonsäure) (HDTMP) und/oder deren Salze;
g) Nitrilotri(methylenphosphonsäure) (NTMP) und/oder deren Salze.

Besonders bevorzugt werden maschinelle Geschirrspülmittel, welche als Phosphonate 1-Hydroxyethan-1,1-diphosphonsäure (HEDP) oder Diethylentriaminpenta(methylenphosphonsäure) (DTPMP) enthalten.

Ferner können die erfindungsgemäßen Geschirrspülmittel, insbesondere maschinellen Geschirrspülmittel, zwei oder mehr unterschiedliche Phosphonate enthalten.

Der Gewichtsanteil der Phosphonate am Gesamtgewicht erfindungsgemäßer Geschirrspülmittel, insbesondere maschineller Geschirrspülmittel, beträgt vorzugsweise 1 bis 8 Gew.-%, vorzugsweise 1,2 bis 6 Gew.-% und insbesondere 1,5 bis 4 Gew.-%.

Erfindungsgemäße Geschirrspülmittel, insbesondere maschinelle Geschirrspülmittel, können ein Tensid oder mehrere Tenside enthalten, wobei insbesondere anionische Tenside, nichtionische Tenside und deren Gemische in Frage kommen.

Unter den anionischen Tensiden bevorzugt sind solche, die mindestens eine Sulfat- oder Sulfonat-Gruppe aufweisen. Das anionische Tensid mit mindestens einer Sulfat- oder Sulfonat-Gruppe ist vorzugsweise ausgewählt aus Fettalkoholsulfaten, Alkansulfonaten und Alkylbenzolsulfonaten. Bevorzugt sind hierbei C₁₂-C₁₈-Fettalkohol-Sulfate (FAS), z.B. Sulfopon K 35 (Cognis, Deutschland), sekundäre C₁₃-C₁₇-Alkansulfonate (SAS), z.B. Hostapur SAS 93 (Clariant, Deutschland), sowie lineare C8-C18-Alkylbenzolsulfonate, insbesondere Dodecylbenzolsulfonat (LAS).

Erfindungsgemäß umfassen die Begriffe "Sulfat" und "Sulfonat" neben betreffenden anionischen Verbindungen, die in Form von Salzen vorliegen, auch die freien Säuren, also die entsprechenden Alkylschwefelsäuren bzw. Alkylsulfonsäuren.

Vorzugsweise ist das anionische Tensid mit mindestens einer Sulfat- oder Sulfonat-Gruppe in erfindungsgemäßen Geschirrspülmitteln in einer Menge von 0,1 bis 20 Gew.-%, besonders bevorzugt, 0,5 bis 15 Gew.-%, insbesondere 2,5 bis 10 Gew.-%, enthalten.

Als nichtionische Tenside können alle dem Fachmann bekannten nichtionischen Tenside eingesetzt werden. Als nichtionische Tenside eignen sich beispielsweise Alkylglykoside der allgemeinen Formel RO(G)ₓ, in der R einem primären geradkettigen oder methylverzweigten, insbesondere in 2-Stellung methylverzweigten aliphatischen Rest mit 8 bis 22, vorzugsweise 12 bis 18 C-Atomen entspricht und G das Symbol ist, das für eine Glykoseeinheit mit 5 oder 6 C-Atomen, vorzugsweise für Glucose, steht. Der Oligomerisierungsgrad x, der die Verteilung von Monoglykosiden und Oligoglykosiden angibt, ist eine beliebige Zahl zwischen 1 und 10; vorzugsweise liegt x bei 1,2 bis 1,4.

Eine weitere Klasse bevorzugt eingesetzter nichtionischer Tenside, die entweder als alleiniges nichtionisches Tensid oder in Kombination mit anderen nichtionischen Tensiden eingesetzt werden, sind alkoxylierte, vorzugsweise ethoxylierte oder ethoxylierte und propoxylierte Fettsäurealkylester, vorzugsweise mit 1 bis 4 Kohlenstoffatomen in der Alkylkette.

Auch nichtionische Tenside vom Typ der Aminoxide, beispielsweise N-Kokosalkyl-N,N-dimethylaminoxid und N-Talgalkyl-N,N-dihydroxyethylaminoxid, und der Fettsäurealkanolamide können geeignet sein. Die Menge dieser nichtionischen Tenside beträgt vorzugsweise nicht mehr als die der ethoxylierten Fettalkohole, insbesondere nicht mehr als die Hälfte davon.

Weitere geeignete Tenside sind Polyhydroxyfettsäureamide der Formel, 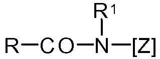 in der R für einen aliphatischen Acylrest mit 6 bis 22 Kohlenstoffatomen, R¹ für Wasserstoff, einen Alkyl- oder Hydroxyalkylrest mit 1 bis 4 Kohlenstoffatomen und [Z] für einen linearen oder verzweigten Polyhydroxyalkylrest mit 3 bis 10 Kohlenstoffatomen und 3 bis 10 Hydroxylgruppen steht. Bei den Polyhydroxyfettsäureamiden handelt es sich um bekannte Stoffe, die üblicherweise durch reduktive Aminierung eines reduzierenden Zuckers mit Ammoniak, einem Alkylamin oder einem Alkanolamin und nachfolgende Acylierung mit einer Fettsäure, einem Fettsäurealkylester oder einem Fettsäurechlorid erhalten werden können.

Zur Gruppe der Polyhydroxyfettsäureamide gehören auch Verbindungen der Formel 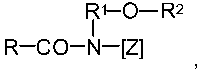 in der R für einen linearen oder verzweigten Alkyl- oder Alkenylrest mit 7 bis 12 Kohlenstoffatomen, R¹ für einen linearen, verzweigten oder zyklischen Alkylrest oder einen Arylrest mit 2 bis 8 Kohlenstoffatomen und R² für einen linearen, verzweigten oder zyklischen Alkylrest oder einen Arylrest oder einen Oxy-Alkylrest mit 1 bis 8 Kohlenstoffatomen steht, wobei C₁₋₄-Alkyl- oder Phenylreste bevorzugt sind und [Z] für einen linearen Polyhydroxyalkylrest steht, dessen Alkylkette mit mindestens zwei Hydroxylgruppen substituiert ist, oder alkoxylierte, vorzugsweise ethoxylierte oder propxylierte Derivate dieses Restes.

[Z] wird vorzugsweise durch reduktive Aminierung eines reduzierten Zuckers erhalten, beispielsweise Glucose, Fructose, Maltose, Lactose, Galactose, Mannose oder Xylose. Die N-Alkoxy- oder N-Aryloxy-substituierten Verbindungen können durch Umsetzung mit Fettsäuremethylestern in Gegenwart eines Alkoxids als Katalysator in die gewünschten Polyhydroxyfettsäureamide überführt werden.

Als bevorzugte Tenside werden schwachschäumende nichtionische Tenside eingesetzt. Mit besonderem Vorzug enthalten Wasch- oder Reinigungsmittel, insbesondere Reinigungsmittel für das Geschirrspülen und hierunter vorzugsweise das maschinelle Geschirrspülen, nichtionische Tenside aus der Gruppe der alkoxylierten Alkohole. Als nichtionische Tenside werden vorzugsweise alkoxylierte, vorteilhafterweise ethoxylierte, insbesondere primäre Alkohole mit vorzugsweise 8 bis 18 C-Atomen und durchschnittlich 1 bis 12 Mol Ethylenoxid (EO) pro Mol Alkohol eingesetzt, in denen der Alkoholrest linear oder bevorzugt in 2-Stellung methylverzweigt sein kann bzw. lineare und methylverzweigte Reste im Gemisch enthalten kann, so wie sie üblicherweise in Oxoalkoholresten vorliegen. Insbesondere sind jedoch Alkoholethoxylate mit linearen Resten aus Alkoholen nativen Ursprungs mit 12 bis 18 C-Atomen, z.B. aus Kokos-, Palm-, Talgfett- oder Oleylalkohol, und durchschnittlich 2 bis 8 Mol EO pro Mol Alkohol bevorzugt. Zu den bevorzugten ethoxylierten Alkoholen gehören beispielsweise C₁₂₋₁₄Alkohole mit 3 EO oder 4 EO, C₉₋₁₁-Alkohol mit 7 EO, C₁₃₋₁₅-Alkohole mit 3 EO, 5 EO, 7 EO oder 8 EO, C₁₂₋₁₈-Alkohole mit 3 EO, 5 EO oder 7 EO und Mischungen aus diesen, wie Mischungen aus C₁₂₋₁₄-Alkohol mit 3 EO und C₁₂₋₁₈-Alkohol mit 5 EO. Die angegebenen Ethoxylierungsgrade stellen statistische Mittelwerte dar, die für ein spezielles Produkt einer ganzen oder einer gebrochenen Zahl entsprechen können. Bevorzugte Alkoholethoxylate weisen eine eingeengte Homologenverteilung auf (narrow range ethoxylates, NRE). Zusätzlich zu diesen nichtionischen Tensiden können auch Fettalkohole mit mehr als 12 EO eingesetzt werden. Beispiele hierfür sind Talgfettalkohol mit 14 EO, 25 EO, 30 EO oder 40 EO.

Mit besonderem Vorzug werden daher ethoxylierte Niotenside, die aus C₆₋₂₀-Monohydroxyalkanolen oder C₆₋₂₀-Alkylphenolen oder C₁₆₋₂₀-Fettalkoholen und mehr als 12 Mol, vorzugsweise mehr als 15 Mol und insbesondere mehr als 20 Mol Ethylenoxid pro Mol Alkohol gewonnen wurden, eingesetzt. Ein besonders bevorzugtes Niotensid wird aus einem geradkettigen Fettalkohol mit 16 bis 20 Kohlenstoffatomen (C₁₆₋₂₀-Alkohol), vorzugsweise einem C₁₈-Alkohol und mindestens 12 Mol, vorzugsweise mindestens 15 Mol und insbesondere mindestens 20 Mol Ethylenoxid gewonnen. Hierunter sind die so genannten "narrow range ethoxylates" besonders bevorzugt.

Mit besonderem Vorzug werden weiterhin Tenside eingesetzt, welche ein oder mehrere Talgfettalkohole mit 20 bis 30 EO in Kombination mit einem Silikonentschäumer enthalten.

Insbesondere bevorzugt sind nichtionische Tenside, die einen Schmelzpunkt oberhalb Raumtemperatur aufweisen. Nichtionische(s) Tensid(e) mit einem Schmelzpunkt oberhalb von 20°C, vorzugsweise oberhalb von 25°C, besonders bevorzugt zwischen 25 und 60°C und insbesondere zwischen 26,6 und 43,3°C, ist/sind besonders bevorzugt.

Geeignete nichtionische Tenside, die Schmelz- bzw. Erweichungspunkte im genannten Temperaturbereich aufweisen, sind beispielsweise schwachschäumende nichtionische Tenside, die bei Raumtemperatur fest oder hochviskos sein können. Werden Niotenside eingesetzt, die bei Raumtemperatur hochviskos sind, so ist bevorzugt, dass diese eine Viskosität oberhalb von 20 Pa·s, vorzugsweise oberhalb von 35 Pa·s und insbesondere oberhalb 40 Pa·s aufweisen. Auch Niotenside, die bei Raumtemperatur wachsartige Konsistenz besitzen, sind bevorzugt.

Niotenside aus der Gruppe der alkoxylierten Alkohole, besonders bevorzugt aus der Gruppe der gemischt alkoxylierten Alkohole und insbesondere aus der Gruppe der EO-AO-EO-Niotenside, werden ebenfalls mit besonderem Vorzug eingesetzt.

Das bei Raumtemperatur feste Niotensid besitzt vorzugsweise Propylenoxideinheiten im Molekül. Vorzugsweise machen solche PO-Einheiten bis zu 25 Gew.-%, besonders bevorzugt bis zu 20 Gew.-% und insbesondere bis zu 15 Gew.-% der gesamten Molmasse des nichtionischen Tensids aus. Besonders bevorzugte nichtionische Tenside sind ethoxylierte Monohydroxyalkanole oder Alkylphenole, die zusätzlich Polyoxyethylen-Polyoxypropylen Blockcopolymereinheiten aufweisen. Der Alkohol- bzw. Alkylphenolteil solcher Niotensidmoleküle macht dabei vorzugsweise mehr als 30 Gew.-%, besonders bevorzugt mehr als 50 Gew.-% und insbesondere mehr als 70 Gew.-% der gesamten Molmasse solcher Niotenside aus. Bevorzugte Mittel sind dadurch gekennzeichnet, dass sie ethoxylierte und propoxylierte Niotenside enthalten, bei denen die Propylenoxideinheiten im Molekül bis zu 25 Gew.-%, bevorzugt bis zu 20 Gew.-% und insbesondere bis zu 15 Gew.-% der gesamten Molmasse des nichtionischen Tensids ausmachen.

Bevorzugt einzusetzende Tenside stammen aus den Gruppen der alkoxylierten Niotenside, insbesondere der ethoxylierten primären Alkohole und Mischungen dieser Tenside mit strukturell komplizierter aufgebauten Tensiden wie Polyoxypropylen/Polyoxyethylen/Polyoxypropylen ((PO/EO/PO)-Tenside). Solche (PO/EO/PO)-Niotenside zeichnen sich darüber hinaus durch gute Schaumkontrolle aus.

Weitere besonders bevorzugt einzusetzende Niotenside mit Schmelzpunkten oberhalb Raumtemperatur enthalten 40 bis 70% eines Polyoxypropylen/Polyoxyethylen/Polyoxypropylen-Blockpolymerblends, der 75 Gew.-% eines umgekehrten Block-Copolymers von Polyoxyethylen und Polyoxypropylen mit 17 Mol Ethylenoxid und 44 Mol Propylenoxid und 25 Gew.-% eines Block-Copolymers von Polyoxyethylen und Polyoxypropylen, initiiert mit Trimethylolpropan und enthaltend 24 Mol Ethylenoxid und 99 Mol Propylenoxid pro Mol Trimethylolpropan, enthält.

Als besonders bevorzugte Niotenside haben sich im Rahmen der vorliegenden Erfindung schwachschäumende Niotenside erwiesen, welche alternierende Ethylenoxid- und Alkylenoxideinheiten aufweisen. Unter diesen sind wiederum Tenside mit EO-AO-EO-AO-Blöcken bevorzugt, wobei jeweils eine bis zehn EO- bzw. AO-Gruppen aneinander gebunden sind, bevor ein Block aus den jeweils anderen Gruppen folgt. Hier sind nichionisches Tenside der allgemeinen Formel 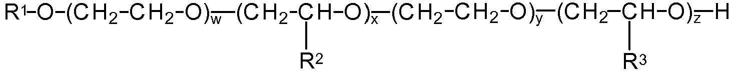 bevorzugt, in der R¹ für einen geradkettigen oder verzweigten, gesättigten oder ein- bzw. mehrfach ungesättigten C₆₋₂₄-Alkyl- oder -Alkenylrest steht; jede Gruppe R² bzw. R³ unabhängig voneinander ausgewählt ist aus -CH₃, -CH₂CH₃, -CH₂CH₂-CH₃, CH(CH₃)₂ und die Indizes w, x, y, z unabhängig voneinander für ganze Zahlen von 1 bis 6 stehen.

Die bevorzugten Niotenside der vorstehenden Formel lassen sich durch bekannte Methoden aus den entsprechenden Alkoholen R¹-OH und Ethylen- bzw. Alkylenoxid herstellen. Der Rest R¹ in der vorstehenden Formel kann je nach Herkunft des Alkohols variieren. Werden native Quellen genutzt, weist der Rest R¹ eine gerade Anzahl von Kohlenstoffatomen auf und ist in der Regel unverzweigt, wobei die linearen Reste aus Alkoholen nativen Ursprungs mit 12 bis 18 C-Atomen, z.B. aus Kokos-, Palm-, Talgfett- oder Oleylalkohol, bevorzugt sind. Aus synthetischen Quellen zugängliche Alkohole sind beispielsweise die Guerbetalkohole oder in 2-Stellung methylverzweigte bzw. lineare und methylverzweigte Reste im Gemisch, so wie sie üblicherweise in Oxoalkoholresten vorliegen. Unabhängig von der Art des zur Herstellung der in den Mitteln enthaltenen Niotenside eingesetzten Alkohols sind Niotenside bevorzugt, bei denen R¹ in der vorstehenden Formel für einen Alkylrest mit 6 bis 24, vorzugsweise 8 bis 20, besonders bevorzugt 9 bis 15 und insbesondere 9 bis 11 Kohlenstoffatomen steht.

Als Alkylenoxideinheit, die alternierend zur Ethylenoxideinheit in den bevorzugten Niotensiden enthalten ist, kommt neben Propylenoxid insbesondere Butylenoxid in Betracht. Aber auch weitere Alkylenoxide, bei denen R² bzw. R³ unabhängig voneinander ausgewählt sind aus -CH₂CH₂-CH₃ bzw. CH(CH₃)₂ sind geeignet. Bevorzugt werden Niotenside der vorstehenden Formel eingesetzt, bei denen R² bzw. R³ für einen Rest -CH₃, w und x unabhängig voneinander für Werte von 3 oder 4 und y und z unabhängig voneinander für Werte von 1 oder 2 stehen.

Zusammenfassend sind insbesondere nichtionische Tenside bevorzugt, die einen C₉₋₁₅-Alkylrest mit 1 bis 4 Ethylenoxideinheiten, gefolgt von 1 bis 4 Propylenoxideinheiten, gefolgt von 1 bis 4 Ethylenoxideinheiten, gefolgt von1 bis 4 Propylenoxideinheiten aufweisen. Diese Tenside weisen in wässriger Lösung die erforderliche niedrige Viskosität auf und sind erfindungsgemäß mit besonderem Vorzug einsetzbar.

Tenside der allgemeinen Formel

R¹-CH(OH)CH₂O-(AO)_{w}-(A'O)ₓ-(A"O)_{y}-(A'''O)_{z}-R²,

in der
R¹ und R² unabhängig voneinander für einen geradkettigen oder verzweigten, gesättigten oder ein- bzw. mehrfach ungesättigten C₂₋₄₀-Alkyl- oder-Alkenylrest steht; A, A', A" und A'" unabhängig voneinander für einen Rest aus der Gruppe -CH₂CH₂, -CH₂CH₂-CH₂, -CH₂-CH(CH₃), -CH₂-CH₂-CH₂-CH₂, -CH₂-CH(CH₃)-CH₂-, -CH₂-CH(CH₂-CH₃) steht; und w, x, y und z für Werte zwischen 0,5 und 90 stehen, wobei x, y und/oder z auch 0 sein können, sind erfindungsgemäß besonders bevorzugt.

Ganz besonders bevorzugt sind hierbei nichtionische Tenside der allgemeinen Formel R¹O[CH₂CH(CH₃)O]ₓ[CH₂CH₂O]_{y}[CH₂CH(CH₃)O]₂CH₂CH(OH)R², in der R¹ für einen linearen oder verzweigten aliphatischen Kohlenwasserstoffrest mit 4 bis 22, insbesondere 6 bis 18, Kohlenstoffatomen oder Mischungen hieraus steht, R² einen linearen oder verzweigten Kohlenwasserstoffrest mit 2 bis 26, insbesondere 4 bis 20, Kohlenstoffatomen oder Mischungen hieraus bezeichnet und x und z für Werte zwischen 0 und 40 und y für einen Wert von mindestens 15, vorzugsweise von 15 bis 120, besonders bevorzugt von 20 bis 80, steht.

In einer bevorzugten Ausführungsform enthält das Geschirrspülmittel, insbesondere das maschinelle Geschirrspülmittel, bezogen auf sein Gesamtgewicht, nichtionisches Tensid der allgemeinen Formel R¹O[CH₂CH(CH₃)O]ₓ[CH₂CH₂O]_{y}[CH₂CH(CH₃)O]₂CH₂CH(OH)R² in Mengen von 0,1 bis 15 Gew.-%, vorzugsweise 0,2 bis 10 Gew.-%, besonders bevorzugt 0,5 bis 8 Gew.-% und insbesondere von 1,0 bis 6 Gew.-%.

Bevorzugt sind insbesondere solche endgruppenverschlossene poly(oxyalkylierten) Niotenside gemäß der Formel R¹O[CH₂CH₂O]_{y}CH₂CH(OH)R², in der R¹ für einen linearen oder verzweigten aliphatischen Kohlenwasserstoffrest mit 4 bis 22, insbesondere 6 bis 16, Kohlenstoffatomen oder Mischungen hieraus steht, R² einen linearen oder verzweigten Kohlenwasserstoffrest mit 2 bis 26, insbesondere 4 bis 20, Kohlenstoffatomen oder Mischungen hieraus bezeichnet und y für einen Wert zwischen 15 und 120 vorzugsweise 20 bis 100, insbesondere 20 bis 80 steht. Zur Gruppe dieser nichtionischen Tenside zählen beispielsweise Hydroxymischether der allgemeinen Formel C₆₋₂₂-CH(OH)CH₂O-(EO)₂₀₋₁₂₀-C₂₋₂₆, zum Beispiel die C₈₋₁₂ Fettalkohol-(EO)₂₂-2-hydroxydecylether und die C₄₋₂₂ Fettalkohol-(EO)₄₀₋₈₀-2-hydroxyalkylether.

Erfindungsgemäße Geschirrspülmittel, insbesondere maschinelle Geschirrspülmittel, dadurch gekennzeichnet, dass als schwachschäumendes nichtionisches Tensid ein Tensid der allgemeinen Formel
R¹CH(OH)CH₂O-(CH₂CH₂O)₂₀₋₁₂₀-R² eingesetzt wird, wobei R¹ und R² unabhängig voneinander für einen linearen oder verzweigten aliphatischen Kohlenwasserstoffrest mit 2 bis 20, insbesondere 4 bis 16, Kohlenstoffatomen stehen, sind besonders bevorzugt.

Bevorzugt sind weiterhin Tenside der Formel R¹O[CH₂CH(CH₃)O]ₓ[CH₂CH₂O]_{y}CH₂CH(OH)R², in der R¹ für einen linearen oder verzweigten aliphatischen Kohlenwasserstoffrest mit 4 bis 22 Kohlenstoffatomen oder Mischungen hieraus steht, R² einen linearen oder verzweigten Kohlenwasserstoffrest mit 2 bis 26 Kohlenstoffatomen oder Mischungen hieraus bezeichnet und x für Werte zwischen 0,5 und 4, vorzugsweise 0,5 bis 1,5, und y für einen Wert von mindestens 15 steht.

Erfindungsgemäß sind weiterhin auch Tenside der allgemeinen Formel
R¹O[CH₂CH(CH₃)O]ₓ[CH₂CH₂O]_{y}CH₂CH(OH)R² bevorzugt, in der R¹ für einen linearen oder verzweigten aliphatischen Kohlenwasserstoffrest mit 4 bis 22 Kohlenstoffatomen oder Mischungen hieraus steht, R² einen linearen oder verzweigten Kohlenwasserstoffrest mit 2 bis 26 Kohlenstoffatomen oder Mischungen hieraus bezeichnet und x für einen Wert zwischen 1 und 40 und y für einen Wert zwischen 15 und 40 steht, wobei die Alkyleneinheiten [CH₂CH(CH₃)O] und [CH₂CH₂O] randomisiert, d.h. in Form einer statistischen, zufälligen Verteilung vorliegen.

Zur Gruppe der bevorzugten endgruppenverschlossene poly(oxyalkylierten) Niotenside zählen auch Niotenside der Formel R¹O[CH₂CH₂O]ₓ[CH₂CH(R³)O]_{y}CH₂CH(OH)R², in der R¹ und R² unabhängig voneinander für einen linearen oder verzweigten, gesättigten oder ein- bzw. mehrfach ungesättigten Kohlenwasserstoffrest mit 2 bis 26 Kohlenstoffatomen steht, R³ unabhängig voneinander ausgewählt ist aus -CH₃, -CH₂CH₃, -CH₂CH₂-CH₃, -CH(CH₃)₂, vorzugsweise jedoch für -CH₃ steht, und x und y unabhängig voneinander für Werte zwischen 1 und 32 stehen, wobei Niotenside mit R³ = -CH₃ und Werten für x von 15 bis 32 und y von 0,5 und 1,5 ganz besonders bevorzugt sind.

Weitere bevorzugt einsetzbare Niotenside sind die endgruppenverschlossenen poly(oxyalkylierten) Niotenside der Formel

R¹O[CH₂CH(R³)O]ₓ[CH₂]ₖCH(OH)[CH₂]ⱼOR²,

in der R¹ und R² für lineare oder verzweigte, gesättigte oder ungesättigte, aliphatische oder aromatische Kohlenwasserstoffreste mit 1 bis 30 Kohlenstoffatomen stehen, R³ für H oder einen Methyl-, Ethyl-, n-Propyl-, iso-Propyl, n-Butyl-, 2-Butyl- oder 2-Methyl-2-Butylrest steht, x für Werte zwischen 1 und 30, k und j für Werte zwischen 1 und 12, vorzugsweise zwischen 1 und 5 stehen.

Wenn der Wert x ≥ 2 ist, kann jedes R³ in der obenstehenden Formel
R¹O[CH₂CH(R³)O]ₓ[CH₂]ₖCH(OH)[CH₂]ⱼOR² unterschiedlich sein. R¹ und R² sind vorzugsweise lineare oder verzweigte, gesättigte oder ungesättigte, aliphatische oder aromatische Kohlenwasserstoffreste mit 6 bis 22 Kohlenstoffatomen, wobei Reste mit 8 bis 18 C-Atomen besonders bevorzugt sind. Für den Rest R³ sind H, -CH₃ oder -CH₂CH₃ besonders bevorzugt. Besonders bevorzugte Werte für x liegen im Bereich von 1 bis 20, insbesondere von 6 bis 15.

Wie vorstehend beschrieben, kann jedes R³ in der obenstehenden Formel unterschiedlich sein, falls x ≥ 2 ist. Hierdurch kann die Alkylenoxideinheit in der eckigen Klammer variiert werden. Steht x beispielsweise für 3, kann der Rest R³ ausgewählt werden, um Ethylenoxid- (R³ = H) oder Propylenoxid- (R³ = CH₃) Einheiten zu bilden, die in jedweder Reihenfolge aneinandergefügt sein können, beispielsweise (EO)(PO)(EO), (EO)(EO)(PO), (EO)(EO)(EO), (PO)(EO)(PO), (PO)(PO)(EO) und (PO)(PO)(PO). Der Wert 3 für x ist hierbei beispielhaft gewählt worden und kann durchaus größer sein, wobei die Variationsbreite mit steigenden x-Werten zunimmt und beispielsweise eine große Anzahl (EO)-Gruppen, kombiniert mit einer geringen Anzahl (PO)-Gruppen einschließt, oder umgekehrt.

Besonders bevorzugte endgruppenverschlossene poly(oxyalkylierte) Alkohole der obenstehenden Formel weisen Werte von k = 1 und j = 1 auf, so dass sich die vorstehende Formel zu

R¹O[CH₂CH(R³)O]ₓCH₂CH(OH)CH₂OR²

vereinfacht. In der letztgenannten Formel sind R¹, R² und R³ wie oben definiert und x steht für Zahlen von 1 bis 30, vorzugsweise von 1 bis 20 und insbesondere von 6 bis 18. Besonders bevorzugt sind Tenside, bei denen die Reste R¹ und R² 9 bis 14 C-Atome aufweisen, R³ für H steht und x Werte von 6 bis 15 annimmt.

Weitere bevorzugt eingesetzte nichtionische Tenside sind nichtionische Tenside der allgemeinen Formel R¹O(AlkO)ₓM(OAlk)_{y}OR², wobei
R¹ und R² unabhängig voneinander für einen verzweigten oder unverzweigten, gesättigten oder ungesättigten, gegebenenfalls hydroxylierten Alkylrest mit 4 bis 22 Kohlenstoffatomen stehen;
Alk für einen verzweigten oder unverzweigten Alkylrest mit 2 bis 4 Kohlenstoffatomen steht;
x und y unabhängig voneinander für Werte zwischen 1 und 70 stehen; und
M für einen Alkylrest aus der Gruppe CH₂, CHR³, CR³R⁴, CH₂CHR³ und CHR³CHR⁴ steht, wobei
R³ und R⁴ unabhängig voneinander für einen verzweigten oder unverzweigten, gesättigten oder ungesättigten Alkylrest mit 1 bis 18 Kohlenstoffatomen stehen.

Bevorzugt sind hierbei nichtionische Tenside der allgemeinen Formel

R¹-CH(OH)CH₂-O(CH₂CH₂O)ₓCH₂CHR(OCH₂CH₂)_{y}O-CH₂CH(OH)-R²,

wobei
- R, R¹ und R² unabhängig voneinander für einen Alkylrest oder Alkenylrest mit 6 bis 22 Kohlenstoffatomen;
- x und y unabhängig voneinander für Werte zwischen 1 und 40 stehen.

Bevorzugt sind hierbei insbesondere Verbindungen der allgemeinen Formel R¹-CH(OH)CH₂-O(CH₂CH₂O)ₓCH₂CHR(OCH₂CH₂)_{y}O-CH₂CH(OH)-R², in denen R für einen linearen, gesättigten Alkylrest mit 8 bis 16 Kohlenstoffatomen, vorzugsweise 10 bis 14 Kohlenstoffatomen steht und n und m unabhängig voneinander Werte von 20 bis 30 aufweisen. Entsprechende Verbindungen können beispielsweise durch Umsetzung von Alkyldiolen HO-CHR-CH₂-OH mit Ethylenoxid erhalten werden, wobei im Anschluss eine Umsetzung mit einem Alkylepoxid zum Verschluss der freien OH-Funktionen unter Ausbildung eines Dihydroxyethers erfolgt.

In einer weiteren bevorzugten Ausführungsform ist das nichtionische Tensid ausgewählt aus nichtionischen Tensiden der allgemeinen Formel

R¹-O(CH₂CH₂O)ₓCR³R⁴(OCH₂CH₂)_{y}O-R²,

in der
- R¹ und R² unabhängig voneinander für einen Alkylrest oder Alkenylrest mit 4 bis 22 Kohlenstoffatomen;
- R³ und R4 unabhängig voneinander für H oder für einen Alkylrest oder Alkenylrest mit 1 bis 18 Kohlenstoffatomen und
- x und y unabhängig voneinander für Werte zwischen 1 und 40 stehen.

Bevorzugt sind hierbei insbesondere Verbindungen der allgemeinen Formel R¹-O(CH₂CH₂O)ₓCR³R⁴(OCH₂CH₂)_{y}O-R², in der R³ und R⁴ für H stehen und die Indices x und y unabhängig voneinander Werte von 1 bis 40, vorzugsweise von 1 bis 15 annehmen.

Besonders bevorzugt sind insbesondere Verbindungen der allgemeinen Formel R¹-O(CH₂CH₂O)ₓCR³R⁴(OCH₂CH₂)_{y}O-R², in der die Reste R¹ und R² unabhängig voneinander gesättigte Alkylreste mit 4 bis 14 Kohlenstoffatome darstellen und die Indices x und y unabhängig voneinander Werte von 1 bis 15 und insbesondere von 1 bis 12 annehmen.

Weiterhin bevorzugt sind solche Verbindungen der allgemeinen Formel R¹-O(CH₂CH₂O)ₓCR³R⁴(OCH₂CH₂)_{y}O-R², in der einer der Reste R¹ und R² verzweigt ist.

Ganz besonders bevorzugt sind Verbindungen der allgemeinen Formel R¹-O(CH₂CH₂O)ₓCR³R⁴(OCH₂CH₂)_{y}O-R², in der die Indices x und y unabhängig voneinander Werte von 8 bis 12 annehmen.

Die angegebenen C-Kettenlängen sowie Ethoxylierungsgrade bzw. Alkoxylierungsgrade der vorgenannten Niotenside stellen statistische Mittelwerte dar, die für ein spezielles Produkt eine ganze oder eine gebrochene Zahl sein können. Aufgrund der Herstellverfahren bestehen Handelsprodukte der genannten Formeln zumeist nicht aus einem individuellen Vertreter, sondern aus Gemischen, wodurch sich sowohl für die C-Kettenlängen als auch für die Ethoxylierungsgrade bzw. Alkoxylierungsgrade Mittelwerte und daraus folgend gebrochene Zahlen ergeben können.

Selbstverständlich können die vorgenannten nichtionischen Tenside nicht nur als Einzelsubstanzen, sondern auch als Tensidgemische aus zwei, drei, vier oder mehr Tensiden eingesetzt werden. Als Tensidgemische werden dabei nicht Mischungen nichtionischer Tenside bezeichnet, die in ihrer Gesamtheit unter eine der oben genannten allgemeinen Formeln fallen, sondern vielmehr solche Mischungen, die zwei, drei, vier oder mehr nichtionische Tenside enthalten, die durch unterschiedliche der vorgenannten allgemeinen Formeln beschrieben werden können.

Insbesondere bevorzugt sind solche nichtionische Tenside, die einen Schmelzpunkt oberhalb Raumtemperatur aufweisen. Nichtionische(s) Tensid(e) mit einem Schmelzpunkt oberhalb von 20°C, vorzugsweise oberhalb von 25°C, besonders bevorzugt zwischen 25 und 60°C und insbesondere zwischen 26,6 und 43,3°C, ist/sind besonders bevorzugt.

Der Gewichtsanteil des nichtionischen Tensids am Gesamtgewicht des erfindungsgemäßen Geschirrspülmittels, insbesondere maschinellen Geschirrspülmittels, beträgt in einer bevorzugten Ausführungsform von 0,1 bis 20 Gew.-%, besonders bevorzugt von 0,5 bis 15 Gew.-%, insbesondere von 2,5 bis 10 Gew.-%.

In einer bevorzugten Ausführungsform beträgt das Gew.-%-Verhältnis von anionischem Tensid mit mindestens einer Sulfat- oder Sulfonat-Gruppe zu nichtionischem Tensid von 3:1 bis 1:3, insbesondere von 2:1 bis 1:2, besonders bevorzugt von 1,5:1 bis 1:1,5.

Erfindungsgemäße Geschirrspülmittel, insbesondere maschinelle Geschirrspülmittel, enthalten als weiteren Bestandteil in einer bevorzugten Ausführungsform mindestens ein anionisches Polymer. Bevorzugte anionische Polymere sind hierbei die copolymeren Polycarboxylate und die copolymeren Polysulfonate.

Der Gewichtsanteil des anionischen Polymers am Gesamtgewicht des erfindungsgemäßen Geschirrspülmittels, insbesondere maschinellen Geschirrspülmittels, beträgt in einer bevorzugten Ausführungsform von 0,1 bis 20 Gew.-%, vorzugsweise von 0,5 bis 18 Gew.-%, besonders bevorzugt von 1,0 bis 15 Gew.-% und insbesondere von 4 bis 14 Gew.-%.

Erfindungsgemäße Geschirrspülmittel, insbesondere maschinelle Geschirrspülmittel, dadurch gekennzeichnet, dass das copolymere anionische Polymer ausgewählt ist aus der Gruppe der hydrophob modifizierten Polycarboxylate und Polysulfonate ist ein besonders bevorzugter Gegenstand, da durch die hydrophobe Modifizierung der anionischen Copolymere eine Verbesserung der Klarspül- und Trocknungseigenschaften dieser Mittel bei gleichzeitig geringer Belagsbildung erreicht werden kann.

Die Copolymere können zwei, drei, vier oder mehr unterschiedliche Monomereinheiten aufweisen.

Bevorzugte copolymere Polysulfonate enthalten neben Sulfonsäuregruppen-haltigem(n) Monomer(en) wenigstens ein Monomer aus der Gruppe der ungesättigten Carbonsäuren.

Als ungesättigte Carbonsäure(n) wird/werden mit besonderem Vorzug ungesättigte Carbonsäuren der Formel R¹(R²)C=C(R³)COOH eingesetzt, in der R¹ bis R³ unabhängig voneinander für -H, -CH₃, einen geradkettigen oder verzweigten gesättigten Alkylrest mit 2 bis 12 Kohlenstoffatomen, einen geradkettigen oder verzweigten, ein- oder mehrfach ungesättigten Alkenylrest mit 2 bis 12 Kohlenstoffatomen, mit -NH₂, -OH oder -COOH substituierte Alkyl- oder Alkenylreste wie vorstehend definiert oder für -COOH oder -COOR⁴ steht, wobei R⁴ ein gesättigter oder ungesättigter, geradkettigter oder verzweigter Kohlenwasserstoffrest mit 1 bis 12 Kohlenstoffatomen ist.

Besonders bevorzugte ungesättigte Carbonsäuren sind Acrylsäure, Methacrylsäure, Ethacrylsäure, α-Chloroacrylsäure, α-Cyanoacrylsäure, Crotonsäure, α-Phenyl-Acrylsäure, Maleinsäure, Maleinsäureanhydrid, Fumarsäure, Itaconsäure, Citraconsäure, Methylenmalonsäure, Sorbinsäure, Zimtsäure oder deren Mischungen. Einsetzbar sind selbstverständlich auch die ungesättigten Dicarbonsäuren.

Als copolymere Polycarboxylate werden erfindungsgemäß besonders bevorzugt Copolymere der Acrylsäure mit Methacrylsäure und der Acrylsäure oder Methacrylsäure mit Maleinsäure eingesetzt. Als besonders geeignet haben sich Copolymere der Acrylsäure mit Maleinsäure erwiesen, die 50 bis 90 Gew.-% Acrylsäure und 50 bis 10 Gew.-% Maleinsäure enthalten. Ihre relative Molekülmasse, bezogen auf freie Säuren, beträgt im allgemeinen 2000 bis 70000 g/mol, vorzugsweise 20000 bis 50000 g/mol und insbesondere 30000 bis 40000 g/mol.

Bei den angegebenen Molmassen handelt es sich im Sinne dieser Schrift um gewichtsmittlere Molmassen Mw, die grundsätzlich mittels Gelpermeationschromatographie (GPC) bestimmt wurden, wobei ein UV-Detektor eingesetzt wurde. Die Messung erfolgte dabei gegen einen externen Standard, der aufgrund seiner strukturellen Verwandtschaft mit den untersuchten Polymeren realistische Molgewichtswerte liefert.

Bei den Sulfonsäuregruppen-haltigen Monomeren sind solche der Formel

R⁵(R⁶)C=C(R⁷)-X-SO₃H

bevorzugt, in der R⁵ bis R⁷ unabhängig voneinander für -H, -CH₃, einen geradkettigen oder verzweigten gesättigten Alkylrest mit 2 bis 12 Kohlenstoffatomen, einen geradkettigen oder verzweigten, ein- oder mehrfach ungesättigten Alkenylrest mit 2 bis 12 Kohlenstoffatomen, mit -NH₂, -OH oder -COOH substituierte Alkyl- oder Alkenylreste oder für -COOH oder -COOR⁴ steht, wobei R⁴ ein gesättigter oder ungesättigter, geradkettigter oder verzweigter Kohlenwasserstoffrest mit 1 bis 12 Kohlenstoffatomen ist, und X für eine optional vorhandene Spacergruppe steht, die ausgewählt ist aus -(CH₂)ₙ- mit n = 0 bis 4, -COO-(CH₂)ₖ- mit k = 1 bis 6, -C(O)-NH-C(CH₃)₂-, -C(O)-NH-C(CH₃)₂-CH₂- und -C(O)-NH-CH(CH₃)-CH₂-.

Unter diesen Monomeren bevorzugt sind solche der Formeln

H₂C=CH-X-SO₃H

H₂C=C(CH₃)-X-SO₃H

HO₃S-X-(R⁶)C=C(R⁷)-X-SO₃H,

in denen R⁶ und R⁷ unabhängig voneinander ausgewählt sind
aus -H, -CH₃, -CH₂CH₃, -CH₂CH₂CH₃ und -CH(CH₃)₂ und X für eine optional vorhandene Spacergruppe steht, die ausgewählt ist aus -(CH₂)ₙ- mit n = 0 bis 4, -COO-(CH₂)ₖ- mit k = 1 bis 6, -C(O)-NH-C(CH₃)₂-, -C(O)-NH-C(CH₃)₂-CH₂- und -C(O)-NH-CH(CH₃)-CH₂-.

Besonders bevorzugte Sulfonsäuregruppen-haltige Monomere sind dabei 1-Acrylamido-1-propansulfonsäure, 2-Acrylamido-2-propansulfonsäure, 2-Acrylamido-2-methyl-1-propansulfonsäure, 2-Methacrylamido-2-methyl-1-propansulfonsäure, 3-Methacrylamido-2-hydroxy-propansulfonsäure, Allylsulfonsäure, Methallylsulfonsäure, Allyloxybenzolsulfonsäure, Methallyloxybenzolsulfonsäure, 2-Hydroxy-3-(2-propenyloxy)propansulfonsäure, 2-Methyl-2-propen1-sulfonsäure, Styrolsulfonsäure, Vinylsulfonsäure, 3-Sulfopropylacrylat, 3-Sulfopropylmethacrylat, Sulfomethacrylamid, Sulfomethylmethacrylamid sowie Mischungen der genannten Säuren oder deren wasserlösliche Salze.

In den Polymeren können die Sulfonsäuregruppen ganz oder teilweise in neutralisierter Form vorliegen, d.h. dass das acide Wasserstoffatom der Sulfonsäuregruppe in einigen oder allen Sulfonsäuregruppen gegen Metallionen, vorzugsweise Alkalimetallionen und insbesondere gegen Natriumionen, ausgetauscht sein kann. Der Einsatz von teil- oder vollneutralisierten sulfonsäuregruppenhaltigen Copolymeren ist erfindungsgemäß bevorzugt.

Die Monomerenverteilung der erfindungsgemäß bevorzugt eingesetzten Copolymere beträgt bei Copolymeren, die nur Carbonsäuregruppen-haltige Monomere und Sulfonsäuregruppen-haltige Monomere enthalten, vorzugsweise jeweils 5 bis 95 Gew.-%, besonders bevorzugt beträgt der Anteil des Sulfonsäuregruppen-haltigen Monomers 50 bis 90 Gew.-% und der Anteil des Carbonsäuregruppen-haltigen Monomers 10 bis 50 Gew.-%, die Monomere sind hierbei vorzugsweise ausgewählt aus den zuvor genannten.

Die Molmasse der erfindungsgemäß bevorzugt eingesetzten Sulfo-Copolymere kann variiert werden, um die Eigenschaften der Polymere dem gewünschten Verwendungszweck anzupassen. Bevorzugte Geschirrspülmittel, insbesondere maschinelle Geschirrspülmittel, sind dadurch gekennzeichnet, dass die Copolymere Molmassen von 2000 bis 200.000 gmol⁻¹, vorzugsweise von 4000 bis 25.000 gmol⁻¹ und insbesondere von 5000 bis 15.000 gmol⁻¹ aufweisen.

In einer weiteren bevorzugten Ausführungsform umfassen die Copolymere neben Carboxylgruppen-haltigem Monomer und Sulfonsäuregruppen-haltigem Monomer weiterhin wenigstens ein nichtionisches, vorzugsweise hydrophobes Monomer. Durch den Einsatz dieser hydrophob modifizierten Polymere konnte insbesondere die Klarspülleistung erfindungsgemäßer maschineller Geschirrspülmittel verbessert werden.

Geschirrspülmittel, insbesondere maschinelle Geschirrspülmittel, dadurch gekennzeichnet, dass das Geschirrspülmittel als anionisches Copolymer ein Copolymer, umfassend
i) Carbonsäuregruppen-haltige Monomer(e)
ii) Sulfonsäuregruppen-haltige Monomer(e)
iii) nichtionische Monomer(e)
enthält, werden erfindungsgemäß bevorzugt.

Als nichtionische Monomere werden vorzugsweise Monomere der allgemeinen Formel R¹(R²)C=C(R³)-X-R⁴ eingesetzt, in der R¹ bis R³ unabhängig voneinander für -H, -CH₃ oder -C₂H₅ steht, X für eine optional vorhandene Spacergruppe steht, die ausgewählt ist aus -CH₂-, -C(O)O- und -C(O)-NH-, und R⁴ für einen geradkettigen oder verzweigten gesättigten Alkylrest mit 2 bis 22 Kohlenstoffatomen oder für einen ungesättigten, vorzugsweise aromatischen Rest mit 6 bis 22 Kohlenstoffatomen steht.

Besonders bevorzugte nichtionische Monomere sind Buten, Isobuten, Penten, 3-Methylbuten, 2-Methylbuten, Cyclopenten, Hexen, Hexen-1, 2-Methlypenten-1, 3-Methlypenten-1, Cyclohexen, Methylcyclopenten, Cyclohepten, Methylcyclohexen, 2,4,4-Trimethylpenten-1, 2,4,4-Trimethylpenten-2, 2,3-Dimethylhexen-1, 2,4-Diemthylhexen-1, 2,5-Dimethlyhexen-1, 3,5-Dimethylhexen-1, 4,4-Dimehtylhexan-1, Ethylcyclohexyn, 1-Octen, α-Olefine mit 10 oder mehr Kohlenstoffatomen wie beispielsweise 1-Decen, 1-Dodecen, 1-Hexadecen, 1-Oktadecen und C22-α-Olefin, 2-Styrol, α-Methylstyrol, 3-Methylstyrol, 4-Propylstryol, 4-Cyclohexylstyrol, 4-Dodecylstyrol, 2-Ethyl-4-Benzylstyrol, 1-Vinylnaphthalin, 2,Vinylnaphthalin, Acrylsäuremethylester, Acrylsäureethylester, Acrylsäurepropylester, Acrylsäurebutylester, Acrylsäurepentylester, Acrylsäurehexylester, Methacrylsäuremethylester, N-(Methyl)acrylamid, Acrylsäure-2-Ethylhexylester, Methacrylsäure-2-Ethylhexylester, *N*-(2-Ethylhexyl)acrylamid, Acrylsäureoctylester, Methacrylsäureoctylester, *N*-(Octyl)acrylamid, Acrylsäurelaurylester, Methacrylsäurelaurylester, *N*-(Lauryl)acrylamid, Acrylsäurestearylester, Methacrylsäurestearylester, *N*-(Stearyl)acrylamid, Acrylsäurebehenylester, Methacrylsäurebehenylester und N-(Behenyl)acrylamid oder deren Mischungen.

In einer weiteren Ausführungsform der Erfindung ist ein erfindungsgemäßes Geschirrspülmittel dadurch gekennzeichnet, dass es mindestens ein weiteres Enzym umfasst, insbesondere eine Protease, Amylase, Cellulase, Pektin-spaltendes Enzym, Hemicellulase, Mannanase, Tannase, Xylanase, Xanthanase, β-Glucosidase, Carrageenase, Perhydrolase, Oxidase, Oxidoreduktase oder eine Lipase, sowie Kombinationen hiervon, insbesondere eine Kombination, die ausgewählt ist aus Protease und Amylase, Protease und Lipase, Protease und Cellulase, Protease und Mannanase, Amylase und Lipase, Amylase und Cellulase, Amylase und Mannanase, Lipase und Cellulase, Lipase und Mannanase, Lipase und Cellulase, Protease und Amylase und Lipase, Protease und Amylase und Cellulase, Protease und Amylase und Mannanase, Amylase und Lipase und Cellulase, Amylase und Lipase und Mannanase, Lipase, Cellulase und Mannanase, Protease und Amylase und Lipase und Cellulase, Protease und Amylase und Cellulase und Mannanase.

Ein derartiges weiteres Enzym ist in dem Mittel vorteilhafterweise jeweils in einer Menge von 1 x 10⁻⁸ bis 5 Gewichts-Prozent bezogen auf aktives Protein enthalten. Zunehmend bevorzugt ist jedes weitere Enzym in einer Menge von 1 x 10⁻⁷-3 Gew.-%, von 0,00001-1 Gew.-%, von 0,00005-0,5 Gew.-%, von 0,0001 bis 0,1 Gew.-% und besonders bevorzugt von 0,0001 bis 0,05 Gew.-% in erfindungsgemäßen Mitteln enthalten, bezogen auf aktives Protein. Die Bestimmung der Aktivproteinkonzentration kann diesbezüglich in fachüblicher Art und Weise erfolgen, bei Hydrolasen beispielsweise über eine Titration der aktiven Zentren unter Verwendung eines geeigneten irreversiblen Inhibitors und Bestimmung der Restaktivität (vgl. beispielsweise M. Bender et al., J. Am. Chem. Soc. 88, 24 (1966), S. 5890-5913; die genannte Referenz betrifft Proteasen, wobei das Prinzip der Titration der aktiven Zentren auf andere Hydrolasen übertragbar ist). Besonders bevorzugt zeigen die Enzyme synergistische Reinigungsleistungen gegenüber bestimmten Anschmutzungen oder Flecken, d.h. die in der Mittelzusammensetzung enthaltenen Enzyme unterstützen sich in ihrer Reinigungsleistung gegenseitig. Ganz besonders bevorzugt liegt ein solches Synergismus vor zwischen der erfindungsgemäß enthaltenen Protease und einem weiteren Enzym eines erfindungsgemäßen Mittels, darunter insbesondere zwischen der erfindungsgemäß enthaltenen Protease und einer Amylase und/oder einer Lipase und/oder einer Mannanase und/oder einer Cellulase und/oder einem Pektin-spaltenden Enzym. Synergistische Effekte können nicht nur zwischen verschiedenen Enzymen, sondern auch zwischen einem oder mehreren Enzymen und weiteren Inhaltsstoffen des erfindungsgemäßen Mittels auftreten.

Unter den Proteasen sind solche vom Subtilisin-Typ bevorzugt. Beispiele hierfür sind die Subtilisine BPN' und Carlsberg, die Protease PB92, die Subtilisine 147 und 309, die Alkalische Protease aus Bacillus lentus, Subtilisin DY und die den Subtilasen, nicht mehr jedoch den Subtilisinen im engeren Sinne zuzuordnenden Enzyme Thermitase, Proteinase K und die Proteasen TW3 und TW7. Subtilisin Carlsberg ist in weiterentwickelter Form unter dem Handelsnamen Alcalase^{®} von der Firma Novozymes A/S, Bagsvaerd, Dänemark, erhältlich. Die Subtilisine 147 und 309 werden unter den Handelsnamen Esperase^{®}, beziehungsweise Savinase^{®} von der Firma Novozymes vertrieben. Von der Protease aus Bacillus lentus DSM 5483 leiten sich die unter der Bezeichnung BLAP^{®} geführten Protease-Varianten ab. Weitere bevorzugte Proteasen sind ferner beispielsweise die unter der Bezeichnung PUR geführten Enzyme. Weitere Proteasen sind ferner die unter den Handelsnamen Durazym^{®}, Relase^{®}, Everlase^{®}, Nafizym^{®}, Natalase^{®}, Kannase^{®} und Ovozyme^{®} von der Firma Novozymes, die unter den Handelsnamen, Purafect^{®}, Purafect^{®} OxP, Purafect^{®} Prime, Excellase^{®} und Properase^{®} von der Firma Genencor, das unter dem Handelsnamen Protosol^{®} von der Firma Advanced Biochemicals Ltd., Thane, Indien, das unter dem Handelsnamen Wuxi^{®} von der Firma Wuxi Snyder Bioproducts Ltd., China, die unter den Handelsnamen Proleather^{®} und Protease P^{®} von der Firma Amano Pharmaceuticals Ltd., Nagoya, Japan, und das unter der Bezeichnung Proteinase K-16 von der Firma Kao Corp., Tokyo, Japan, erhältlichen Enzyme. Besonders bevorzugt eingesetzt werden auch die Proteasen aus Bacillus gibsonii und Bacillus pumilus, die offenbart sind in den internationalen Patentanmeldungen WO2008/086916 und WO2007/131656.

Erfindungsgemäß konfektionierbare Amylasen sind beispielsweise die α-Amylasen aus Bacillus licheniformis, aus Bacillus amyloliquefaciens oder aus Bacillus stearothermophilus sowie insbesondere auch deren für den Einsatz in Wasch- oder Reinigungsmitteln verbesserte Weiterentwicklungen. Das Enzym aus Bacillus licheniformis ist von dem Unternehmen Novozymes unter dem Namen Termamyl^{®} und von dem Unternehmen Danisco/Genencor unter dem Namen Purastar^{®}ST erhältlich. Weiterentwicklungsprodukte dieser α-Amylase sind von dem Unternehmen Novozymes unter den Handelsnamen Duramyl^{®} und Termamy^{®}ultra, von dem Unternehmen Danisco/Genencor unter dem Namen Purastar^{®}OxAm und von dem Unternehmen Daiwa Seiko Inc., Tokyo, Japan, als Keistase^{®} erhältlich. Die α-Amylase von Bacillus amyloliquefaciens wird von dem Unternehmen Novozymes unter dem Namen BAN^{®} vertrieben, und abgeleitete Varianten von der α-Amylase aus Bacillus stearothermophilus unter den Namen BSG^{®} und Novamyl^{®}, ebenfalls von dem Unternehmen Novozymes. Des Weiteren sind für diesen Zweck die α-Amylase aus Bacillus sp. A 7-7 (DSM 12368) und die Cyclodextrin-Glucanotransferase (CGTase) aus Bacillus agaradherens (DSM 9948) hervorzuheben. Ebenso sind Fusionsprodukte aller genannten Moleküle einsetzbar. Darüber hinaus sind die unter den Handelsnamen Fungamyl^{®} von dem Unternehmen Novozymes erhältlichen Weiterentwicklungen der α-Amylase aus Aspergillus niger und A. oryzae geeignet. Weitere vorteilhaft einsetzbare Handelsprodukte sind beispielsweise die Amylase-LT^{®} und Stainzyme^{®} oder Stainzyme ultra^{®} bzw. Stainzyme plus^{®}, letztere ebenfalls von dem Unternehmen Novozymes. Auch durch Punktmutationen erhältliche Varianten dieser Enzyme können erfindungsgemäß eingesetzt werden. Besonders bevorzugte Amylasen sind offenbart in den internationalen Offenlegungsschriften WO 00/60060, WO 03/002711, WO 03/054177 und WO07/079938, auf deren Offenbarung daher ausdrücklich verwiesen wird bzw. deren diesbezüglicher Offenbarungsgehalt daher ausdrücklich in die vorliegende Patentanmeldung mit einbezogen wird. Erfindungsgemäß konfektionierbare Amylasen sind ferner vorzugsweise α-Amylasen.

Beispiele für erfindungsgemäß konfektionierbare Lipasen oder Cutinasen, die insbesondere wegen ihrer Triglycerid-spaltenden Aktivitäten enthalten sind, aber auch, um aus geeigneten Vorstufen in situ Persäuren zu erzeugen, sind die ursprünglich aus Humicola lanuginosa (Thermomyces lanuginosus) erhältlichen, beziehungsweise weiterentwickelten Lipasen, insbesondere solche mit dem Aminosäureaustausch D96L. Sie werden beispielsweise von der Firma Novozymes unter den Handelsnamen Lipolase^{®}, Lipolase^{®}Ultra, LipoPrime^{®}, Lipozyme^{®} und Lipex^{®} vertrieben. Desweiteren sind beispielsweise die Cutinasen einsetzbar, die ursprünglich aus Fusarium solani pisi und Humicola insolens isoliert worden sind. Von der Firma Genencor sind beispielsweise die Lipasen beziehungsweise Cutinasen einsetzbar, deren Ausgangsenzyme ursprünglich aus Pseudomonas mendocina und Fusarium solanii isoliert worden sind. Als weitere wichtige Handelsprodukte sind die ursprünglich von der Firma Gist-Brocades vertriebenen Präparationen M1 Lipase^{®} und Lipomax^{®} und die von der Firma Meito Sangyo KK, Japan, unter den Namen Lipase MY-30^{®}, Lipase OF^{®} und Lipase PL^{®} vertriebenen Enzyme zu erwähnen, ferner das Produkt Lumafast^{®} von der Firma Genencor.

Erfindungsgemäß konfektionierbare Cellulasen (Endoglucanasen, EG) umfassen beispielsweise die pilzliche, Endoglucanase(EG)-reiche Cellulase-Präparation beziehungsweise deren Weiterentwicklungen, die von dem Unternehmen Novozymes unter dem Handelsnamen Celluzyme^{®} angeboten wird. Die ebenfalls von dem Unternehmen Novozymes erhältlichen Produkte Endolase^{®} und Carezyme^{®} basieren auf der 50 kD-EG, beziehungsweise der 43 kD-EG aus Humicola insolens DSM 1800. Weitere einsetzbare Handelsprodukte dieses Unternehmens sind Cellusoft^{®}, Renozyme^{®} und Celluclean^{®}. Weiterhin einsetzbar sind beispielsweise Cellulasen, die von dem Unternehmen AB Enzymes, Finnland, unter den Handelsnamen Ecostone^{®} und Biotouch^{®} erhältlich sind, und die zumindest zum Teil auf der 20 kD-EG aus Melanocarpus basieren. Weitere Cellulasen von dem Unternehmen AB Enzymes sind Econase^{®} und Ecopulp^{®}. Weitere geeignete Cellulasen sind aus Bacillus sp. CBS 670.93 und CBS 669.93, wobei die aus Bacillus sp. CBS 670.93 von dem Unternehmen Danisco/Genencor unter dem Handelsnamen Puradax^{®} erhältlich ist. Weitere verwendbare Handelsprodukte des Unternehmens Danisco/Genencor sind "Genencor detergent cellulase L" und Indi-Age^{®}Neutra.

Auch durch Punktmutationen erhältliche Varianten dieser Enzyme können erfindungsgemäß eingesetzt werden. Besonders bevorzugte Cellulasen sind Thielavia terrestris Cellulasevarianten, die in der internationalen Offenlegungsschrift WO 98/12307 offenbart sind, Cellulasen aus Melanocarpus, insbesondere Melanocarpus albomyces, die in der internationalen Offenlegungsschrift WO 97/14804 offenbart sind, Cellulasen vom EGIII-Typ aus Trichoderma reesei, die in der europäischen Patentanmeldung EP 1 305 432 offenbart sind bzw. hieraus erhältliche Varianten, insbesondere diejenigen, die offenbart sind in den europäischen Patentanmeldungen EP 1240525 und EP 1305432, sowie Cellulasen, die offenbart sind in den internationalen Offenlegungsschriften WO 1992006165, WO 96/29397 und WO 02/099091. Auf deren jeweilige Offenbarung wird daher ausdrücklich verwiesen bzw. deren diesbezüglicher Offenbarungsgehalt wird daher ausdrücklich in die vorliegende Patentanmeldung mit einbezogen.

Pektin-spaltende Enzyme (Pektinasen) im Sinne der Erfindung sind Enzyme, die Pektine und/oder andere Galakturonane spalten. Bei Pektinen handelt es sich um Polysaccharide, deren Hauptbestandteil α-D-Galacturonsäure als Monomer ist, vorzugsweise zu mindestens 50 Gew.-% und besonders bevorzugt zu mindestens 65 Gew.-%. Diese Galacturonsäure-Monomere sind über α-1,4-, manchmal auch zu einem geringen Anteil über β-1,4-glycosidische Bindungen, miteinander verbunden und bilden das Rückgrat des Pektinmoleküls, das periodisch durch 1,2-Bindungen mit α-L-Rhamnose unterbrochen wird. Ein Pektin ist folglich eine Rhamno-galacturonsäure. Eine Pektin-spaltendes Enzym ist folglich insbesondere ein Enzym, das die Hydrolyse von 1,4-α-D-galaktosiduronischen Bindungen katalysiert.

Innerhalb der EC-Klassifikation der Enzyme, dem numerischen Klassifikationssystem für Enzyme, sind die Pektin-spaltenden Enzyme insbesondere zugehörig zu den Enzymklassen (engl. "Enzyme Commission number") EC 3.1.1.11, EC 3.2.1.15, EC 3.2.1.67 und EC 3.2.1.82 und zählen folglich zur dritten der sechs Enzymhauptklassen, den Hydrolasen (E.C.3.-.-.-), hierunter zu den Glycosylasen (E.C. 3.2.-.-) und wiederum hierunter zu den Glycosidasen (E.C. 3.2.1.-), d.h.

Enzymen, die O- und/oder S-Glycosyl-Verbindungen hydrolysieren. Pektin-spaltende Enyzme wirken folglich insbesondere gegen Rückstände auf Geschirr, die Pektinsäure und/oder andere Galakturonane enthalten, und katalysieren deren Hydrolyse.

Zu den Pektin-spaltenden Enzymen werden im Rahmen der vorliegenden Erfindung ebenfalls Enzyme gezählt mit den Bezeichnungen Pektinase, Pektatlyase, Pektinesterase, Pektindemethoxylase, Pektinmethoxylase, Pektinmethylesterase, Pektase, Pektinmethylesterase, Pektinoesterase, Pektinpektylhydrolase, Pektindepolymerase, Endopolygalacturonase, Pektolase, Pektinhydrolase, Pektin-Polygalacturonase, Endo-Polygalacturonase, Poly-α-1,4-Galacturonid Glycanohydrolase, Endogalacturonase, Endo-D-galacturonase, Galacturan 1,4-α-Galacturonidase, Exopolygalacturonase, Poly(galacturonat) Hydrolase, Exo-D-Galacturonase, Exo-D-Galacturonanase, Exopoly-D-Galacturonase, Exo-poly-α-Galacturonosidase, Exopolygalacturonosidase oder Exopolygalacturanosidase.

Beispiele für diesbezüglich geeignete Enzyme sind beispielsweise unter den Namen Gamanase^{®}, Pektinex AR^{®}, X-Pect^{®} oder Pectaway^{®} von dem Unternehmen Novozymes, unter dem Namen Rohapect UF^{®}, Rohapect TPL^{®}, Rohapect PTE100^{®}, Rohapect MPE^{®}, Rohapect MA plus HC, Rohapect DA12L^{®}, Rohapect 10L^{®}, Rohapect B1L^{®} von dem Unternehmen AB Enzymes und unter dem Namen Pyrolase^{®} von dem Unternehmen Diversa Corp., San Diego, CA, USA erhältlich.

Ferner können insbesondere zur Entfernung bestimmter Problemanschmutzungen weitere Enzyme eingesetzt sein, die unter dem Begriff Hemicellulasen zusammengefasst werden. Hierzu gehören beispielsweise Mannanasen, Xanthanlyasen, Xanthanasen, Xyloglucanasen, Xylanasen, Pullulanasen und β-Glucanasen. Die aus Bacillus subtilis gewonnene β-Glucanase ist unter dem Namen Cereflo^{®} von der Firma Novozymes erhältlich. Erfindungsgemäß besonders bevorzugte Hemicellulasen sind Mannanasen, welche beispielsweise unter den Handelsnamen Mannaway^{®} von dem Unternehmen Novozymes oder Purabrite^{®} von dem Unternehmen Genencor vertrieben werden.

Zur Erhöhung der bleichenden Wirkung kann ein erfindungsgemäßes Geschirrspülmittel auch Oxidoreduktasen, beispielsweise Oxidasen, Oxygenasen, Katalasen (die bei niedrigen H₂O₂-Konzentrationen als Peroxidase reagieren), Peroxidasen, wie Halo-, Chloro-, Bromo-, Lignin-, Glucose- oder Manganperoxidasen, Dioxygenasen oder Laccasen (Phenoloxidasen, Polyphenoloxidasen) enthalten. Als geeignete Handelsprodukte sind Denilite^{®} 1 und 2 der Firma Novozymes zu nennen. Als vorteilhaft einsetzbare Beispielsysteme für eine enzymatische Perhydrolyse wird auf die Anmeldungen WO 98/45398 A1, WO 2005/056782 A2 sowie WO 2004/058961 A1 verwiesen. Ein kombiniertes enzymatisches Bleichsystem, umfassend eine Oxidase und eine Perhydrolase beschreibt die Anmeldung WO 2005/124012. Vorteilhafterweise werden zusätzlich vorzugsweise organische, besonders bevorzugt aromatische, mit den Enzymen wechselwirkende Verbindungen zugegeben, um die Aktivität der betreffenden Oxidoreduktasen zu verstärken (Enhancer) oder um bei stark unterschiedlichen Redoxpotentialen zwischen den oxidierenden Enzymen und den Anschmutzungen den Elektronenfluß zu gewährleisten (Mediatoren).

Die erfindungsgemäß einzusetzenden Enzyme können ferner zusammen mit Begleitstoffen, etwa aus der Fermentation, oder mit Stabilisatoren konfektioniert sein und in einer derartigen Konfektionierungsform in ein erfindungsgemäßes Geschirrspülmittel eingearbeitet werden.

Die zuvor beschriebenen Wirkstoffkombinationen eignen sich insbesondere zur Entfernung bleichbarer Anschmutzungen, insbesondere Teeanschmutzungen, in Geschirrspülverfahren, insbesondere in maschinellen Geschirrspülverfahren.

Ein weiterer Gegenstand der Erfindung ist daher ein Verfahren zur Entfernung von Anschmutzungen, insbesondere Teeanschmutzungen, auf harten Oberflächen, insbesondere Geschirr, umfassend einen der Verfahrensschritte
(a) In Kontakt bringen der harten Oberfläche mit einer Reinigungsflotte, die ein erfindungsgemäßes Geschirrspülmittel enthält, oder
(b) In Kontakt bringen der harten Oberfläche mit einer Reinigungsflotte, die eine Wasserstoffperoxidquelle, einen Bleichkatalysator und eine Protease, wobei die Protease eine Aminosäuresequenz umfasst, die zu der in SEQ ID NO. 3 angegebenen Aminosäuresequenz identisch ist, wobei der Bleichkatalysator ausgewählt ist aus der Gruppe der Komplexe des Mangans mit 1,4,7-trimethyl-1,4,7-triazacyclononan (Me-TACN) oder 1,2,4,7-tetramethyl-1,4,7-triazacyclononan (Me/Me-TACN) und wobei die Wasserstoffperoxidquelle Natriumpercarbonat ist.

Vorzugsweise handelt es sich um ein maschinelles Geschirrspülverfahren. Das Geschirrspülmittel wird vorzugsweise während des Durchlaufens eines Geschirrspülprogramms, vor Beginn des Hauptspülgangs oder im Verlaufe des Hauptspülgangs in den Innenraum einer Geschirrspülmaschine eindosiert. Die Eindosierung bzw. der Eintrag des erfindungsgemäßen Mittels in den Innenraum der Geschirrspülmaschine kann manuell erfolgen, vorzugsweise wird das Mittel jedoch mittels der Dosierkammer der Geschirrspülmaschine in den Innenraum der Geschirrspülmaschine dosiert. Im Verlauf des Reinigungsverfahrens wird vorzugsweise kein zusätzlicher Wasserenthärter und kein zusätzlicher Klarspüler in den Innenraum der Geschirrspülmaschine dosiert. Vorzugsweise handelt es sich um ein Verfahren zur Entfernung bleichbarer Anschmutzungen.

Alle Sachverhalte, Gegenstände und Ausführungsformen, die für erfindungsgemäße Geschirrspülmittel beschrieben sind, auch auf erfindungsgemäße Verfahren anwendbar. Daher wird an dieser Stelle ausdrücklich auf die Offenbarung an entsprechender Stelle verwiesen mit dem Hinweis, dass diese Offenbarung auch für die vorstehenden erfindungsgemäßen Verfahren gilt.

Ein weiterer Gegenstand der Erfindung ist die Verwendung eines erfindungsgemäßen Geschirrspülmittels zur Entfernung von Anschmutzungen, insbesondere Teeanschmutzungen, auf harten Oberflächen, oder einer Protease, die eine Aminosäuresequenz umfasst, die zu der in SEQ ID NO. 3 angegebenen Aminosäuresequenz identisch ist, zur Entfernung von Anschmutzungen, insbesondere Teeanschmutzungen, auf harten Oberflächen in einer Reinigungsflotte, die ferner eine Wasserstoffperoxidquelle und einen Bleichkatalysator umfasst, wobei der Bleichkatalysator ausgewählt ist aus der Gruppe der Komplexe des Mangans mit 1,4,7-trimethyl-1,4,7-triazacyclononan (Me-TACN) oder 1,2,4,7-tetramethyl-1,4,7-triazacyclononan (Me/Me-TACN) und wobei die Wasserstoffperoxidquelle Natriumpercarbonat ist.
Vorzugsweise betrifft die Verwendung die Entfernung bleichbarer Anschmutzungen. Alle Sachverhalte, Gegenstände und Ausführungsformen, die für erfindungsgemäße Geschirrspülmittel bzw. erfindungsgemäße Verfahren beschrieben sind, sind auch auf die genannten Verwendungen anwendbar. Daher wird an dieser Stelle ausdrücklich auf die Offenbarung an entsprechender Stelle verwiesen mit dem Hinweis, dass diese Offenbarung auch für die vorstehenden erfindungsgemäßen Verwendungen gilt.

### Beispiele

### Beispiel 1: Ermittlung der Migrationsweite von Proteasen

Von vier Proteasen wurde die Migrationsweite gegenüber der Protease gemäß SEQ ID NO. 1 in einer nativen Polyacrylamidgelelektrophorese bestimmt. Hierfür wurde das PHASTSystem der Firma GE Healthcare verwendet. Als Trenngel benutzt wurden native "PhastGel Gradient 8-25"-Gele aus Polyacrylamid, die eine Größe von 43 x 50 x 0,45 mm aufwiesen, in Kombination mit einem 0,112M Tris, 0,112M Acetat (pH 6,4) Puffer, denen "Native Buffer Strips" aus 3% Agarose als Sammelgel vorgelagert waren, die eine Größe von 41 x 10 x 6 mm aufwiesen, in Kombination mit einem 0,25M Tris, 0,88M L-Alanin (pH 8,8) Puffer. Die Auftrennung erfolgte nach Angaben des Herstellers wie in "PhastSystem Separation Technique File No. 120" (Amersham Biosciences 1998) beschrieben, jedoch im "reversed polarity electrode mode" zur Auftrennung basischer Proteine. Die Elektrophorese erfolgte so lange, bis die Proteasebanden, mindestens die Hälfte der zur Verfügung stehenden Laufstrecke in dem Trenngel zurückgelegt hatten.

Die verwendeten Proteasen sowie deren Migrationsweiten sind in nachfolgender Tabelle 1 dargestellt. Ferner ist der berechnete isolelektrische Punkt bei pH 7 und die berechnete Nettoladung bei pH 7 für die jeweilige Protease angegeben. Die Berechnung des isoelektrischen Punktes und der Nettoladung erfolgten mittels der "Analysis"-Funktion des BioAnnotater-Moduls des Softwarepakets Vector NTI^{®} Advance 10.3.0 (Invitrogen Corporation, 1600 Faraday Avenue, Carlsbad, Kalifornien, USA) mit den vorgegebenen Standard (Default)-Parametern.

**Tabelle 1:**

| | | Migrationsweite im Vergleich zur Migrationsweite der Protease gemäß SEQ ID NO. 1 (angegeben als Faktor; -fach) | isoelektrischer Punkt bei pH7 (berechnet) | Nettoladung bei pH 7 (berechnet) |
|---|---|---|---|---|
| Protease 1 | Protease Ovozyme^{®} 64T (Novozymes) | 0,9 | 8,97 | 2,38 |
| Protease 2 | leistungsverbesserte Variante F49 der Protease aus Bacillus lentus gemäß WO 95/23221 | 0,9 | 8,97 | 2,38 |
| Protease 3 | Protease gemäß SEQ ID NO. 2 | 1,2 | 9,69 | 5,38 |
| Protease 4 | Protease gemäß SEQ ID NO. 3 | 1,1 | 9,52 | 4,38 |
| Protease-Referenz | Protease gemäß SEQ ID NO. 1 | 1 | 9,30 | 3,38 |

### Beispiel 2: Ermittlung der Reinigungsleistung erfindungsgemäßer Geschirrspülmittel

Es wurde die Reinigungsleistung an Teeanschmutzungen eines handelsüblichen automatischen Geschirrspülmittels in Form einer Geschirrspülmitteltablette, das 15 Gew.-% Natriumpercarbonat als Wasserstoffperoxidquelle (Bleichmittel), 0,03 Gew.-% Mn-Me-TACN (Mn-TACN) als Bleichkatalysator und 2,2 Gew.-% TAED als Bleichaktivator enthielt, getestet, dem jeweils Proteasegranulate mit unterschiedlichen Proteasen gemäß Beispiel 1 zugesetzt wurden. Die Proteasen wurden gesamtproteingleich (0,056 g Protease pro Spülvorgang) eingesetzt. Das Geschirrspülverfahren wurde in der Spülmaschine Miele G698SC (Programm: 50°C, Programmdauer 57 min, Wasserhärte 21 °deutscher Härte) durchgeführt. Die Geschirrspülmitteltablette wurde vor Beginn des Reinigungsprogramms in die Dosiervorrichtung gegeben. Es wurden jeweils 3 Bestimmungen durchgeführt. Jeder Versuch wurde gezählt und am Ende der Mittelwert gebildet. Die Auswertung der Reinigungsleistung erfolgte visuell gemäß einer Skala von 1 bis 10, wobei der Wert 10 die beste Note ist (kein erkennbarer Rückstand). Die Ergebnisse sind in den nachfolgenden Tabellen 2a (Versuchsreihe 1) und 2b (Versuchsreihe 2) dargestellt.

**Tabelle 2a:**

| Geschirrspülmitteltablette, die Natriumpercarbonat und Mn-TACN enthält | Tee |
|---|---|
| ohne Protease | 6,5 |
| mit Protease 1 | 5,5 |
| mit Protease 2 | 5,8 |
| mit Protease 4 | 8,7 |

**Tabelle 2b:**

| Geschirrspülmitteltablette, die Natriumpercarbonat und Mn-TACN enthält | Tee |
|---|---|
| ohne Protease | 5,1 |
| mit Protease 3 | 6,3 |

Es wird deutlich, dass die erfindungsgemäßen Geschirrspülmittel mit den Proteasen 3 und 4 eine signifikant (mehr als eine Note) verbesserte Reinigungsleistung an Teeanschmutzungen zeigen.

### Beispiel 3: Ermittlung der Reinigungsleistung erfindungsgemäßer Geschirrspülmittel

Es wurde die Reinigungsleistung an Teeanschmutzungen eines handelsüblichen automatischen Geschirrspülmittels in Form einer Geschirrspülmitteltablette, das 15 Gew.-% Natriumpercarbonat als Wasserstoffperoxidquelle (Bleichmittel), 2,2 Gew.-% TAED als Bleichaktivator und gegebenenfalls 0,03 Gew.-% Mn-Me-TACN (Mn-TACN) als Bleichkatalysator enthielt, getestet, dem jeweils Proteasegranulate mit unterschiedlichen Proteasen gemäß Beispiel 1 zugesetzt wurden. Die Proteasen wurden wieder gesamtproteingleich (0,056 g Protease pro Spülvorgang) eingesetzt. Die weitere Zusammensetzung der Geschirrspülmitteltablette entsprach derjenigen aus Beispiel 2. Die Durchführung und Auswertung erfolgte wie in Beispiel 2 beschrieben. Die Ergebnisse sind in der nachfolgenden Tabelle 3 dargestellt.

**Tabelle 3:**

| Geschirrspülmitteltablette, die Natriumpercarbonat enthält | Tee |
|---|---|
| Mit Protease 1 ohne Mn-TACN | 4,8 |
| Mit Protease 1 mit Mn-TACN | 5,7 |
| Mit Protease 4 ohne Mn-TACN | 5,1 |
| Mit Protease 4 mit Mn-TACN | 9,1 |

Es wird wiederum deutlich, dass die erfindungsgemäßen Geschirrspülmittel mit der Protease 4 eine signifikant verbesserte Reinigungsleistung an Teeanschmutzungen in Kombination mit dem Bleichkatalysator Mn-TACN zeigen.

## Patentansprüche

1. Geschirrspülmittel umfassend eine Wasserstoffperoxidquelle, einen Bleichkatalysator und eine Protease, **dadurch gekennzeichnet, dass**
der Bleichkatalysator ausgewählt ist aus der Gruppe der Komplexe des Mangans mit 1,4,7-trimethyl-1,4,7-triazacyclononan (Me-TACN) oder 1,2,4,7-tetramethyl-1,4,7-triazacyclononan (Me/Me-TACN),
die Protease eine Aminosäuresequenz umfasst, die zu der in SEQ ID NO. 3 angegebenen Aminosäuresequenz identisch ist und
die Wasserstoffperoxidquelle Natriumpercarbonat ist.

2. Geschirrspülmittel nach Anspruch 1, **dadurch gekennzeichnet, dass** es
die Protease in einer Menge von 1 x 10⁻⁸-10 Gew.-%, bezogen auf den Gesamtproteingehalt der Protease, enthält, und/oder
den Bleichkatalysator in einer Menge von 0,0025-1 Gew.-% enthält, und/oder
die Wasserstoffperoxidquelle in einer Menge von 2-30 Gew.-% enthält.

3. Geschirrspülmittel nach einem der Ansprüche 1 bis 2, **dadurch gekennzeichnet, dass** es ferner einen Bleichaktivator umfasst, insbesondere TAED, vorzugsweise in einer Menge von 0,1-10 Gew.-%.

4. Geschirrspülmittel nach einem der Ansprüche 1 bis 3, **dadurch gekennzeichnet, dass** es sich um ein maschinelles Geschirrspülmittel handelt.

5. Geschirrspülmittel nach einem der Ansprüche 1 bis 4, **dadurch gekennzeichnet, dass** es in fester Form vorliegt, insbesondere als rieselfähiges Pulver oder als Formkörper, oder dass es in flüssiger, gelförmiger oder pastöser Form vorliegt.

6. Geschirrspülmittel nach einem der Ansprüche 1 bis 5, **dadurch gekennzeichnet, dass** es mindestens einen weiteren Inhaltsstoff umfasst, der ausgewählt ist aus der Gruppe bestehend aus Gerüststoff, Tensid, anionisches Polymer sowie Kombinationen hiervon, und/oder dass es mindestens ein weiteres Enzym umfasst, insbesondere eine Protease, Amylase, Cellulase, Pektin-spaltendes Enzym, Hemicellulase, Mannanase, Tannase, Xylanase, Xanthanase, β-Glucosidase, Carrageenase, Perhydrolase, Oxidase, Oxidoreduktase oder eine Lipase, sowie Kombinationen hiervon, insbesondere eine Kombination, die ausgewählt ist aus Protease und Amylase, Protease und Lipase, Protease und Cellulase, Protease und Mannanase, Amylase und Lipase, Amylase und Cellulase, Amylase und Mannanase, Lipase und Cellulase, Lipase und Mannanase, Lipase und Cellulase, Protease und Amylase und Lipase, Protease und Amylase und Cellulase, Protease und Amylase und Mannanase, Amylase und Lipase und Cellulase, Amylase und Lipase und Mannanase, Lipase, Cellulase und Mannanase, Protease und Amylase und Lipase und Cellulase, Protease und Amylase und Cellulase und Mannanase.

7. Verfahren zur Entfernung von Anschmutzungen, insbesondere Teeanschmutzungen, auf harten Oberflächen, insbesondere Geschirr, umfassend einen der Verfahrensschritte
(a) In Kontakt bringen der harten Oberfläche mit einer Reinigungsflotte, die ein Geschirrspülmittel gemäß einem der Ansprüche 1 bis 6 enthält, oder
(b) In Kontakt bringen der harten Oberfläche mit einer Reinigungsflotte, die eine Wasserstoffperoxidquelle, einen Bleichkatalysator und eine Protease umfasst, wobei die Protease eine Aminosäuresequenz umfasst, die zu der in SEQ ID NO. 3 angegebenen Aminosäuresequenz identisch ist, wobei der Bleichkatalysator ausgewählt ist aus der Gruppe der Komplexe des Mangans mit 1,4,7-trimethyl-1,4,7-triazacyclononan (Me-TACN) oder 1,2,4,7-tetramethyl-1,4,7-triazacyclononan (Me/Me-TACN) und wobei die Wasserstoffperoxidquelle Natriumpercarbonat ist.

8. Verwendung eines Geschirrspülmittels nach einem der Ansprüche 1 bis 6 zur Entfernung von Anschmutzungen, insbesondere Teeanschmutzungen, auf harten Oberflächen, oder einer Protease, die eine Aminosäuresequenz umfasst, die zu der in SEQ ID NO. 3 angegebenen Aminosäuresequenz identisch ist, zur Entfernung von Anschmutzungen, insbesondere Teeanschmutzungen, auf harten Oberflächen in einer Reinigungsflotte, wobei die Reinigungsflotte ferner eine Wasserstoffperoxidquelle und einen Bleichkatalysator umfasst, wobei der Bleichkatalysator ausgewählt ist aus der Gruppe der Komplexe des Mangans mit 1,4,7-trimethyl-1,4,7-triazacyclononan (Me-TACN) oder 1,2,4,7-tetramethyl-1,4,7-triazacyclononan (Me/Me-TACN) und wobei die Wasserstoffperoxidquelle Natriumpercarbonat ist.

## Claims

1. Dishwashing detergent comprising a hydrogen peroxide source, a bleaching catalyst, and a protease, **characterized in that**
the bleaching catalyst is selected from the group consisting of complexes of manganese with 1,4,7-trimethyl-1,4,7-triazacyclononane (Me-TACN) or 1,2,4,7-tetramethyl-1,4,7-triazacyclononane (Me/Me-TACN),
the protease comprises an amino acid sequence that is identical to the amino acid sequence specified in SEQ ID NO. 3, and
the hydrogen peroxide source is sodium percarbonate.

2. Dishwashing detergent according to claim 1, **characterized in that** it
the protease is present in an amount of 1 x 10⁻⁸-10 wt.%, based on the total protein content of the protease, and/or
the bleach catalyst in an amount of 0.0025-1 wt.%, and/or
the hydrogen peroxide source in an amount of 2-30 wt.%.

3. Dishwashing detergent according to one of claims 1 to 2, **characterized in that** it further comprises a bleach activator, in particular TAED, preferably in an amount of 0.1-10 wt.%.

4. Dishwashing detergent according to any one of claims 1 to 3, **characterized in that** it is a machine dishwashing detergent.

5. Dishwashing detergent according to one of claims 1 to 4, **characterized in that** it is in solid form, in particular as a free-flowing powder or as a molded body, or **in that** it is in liquid, gel or paste form.

6. Dishwashing detergent according to one of claims 1 to 5, **characterized in that** it comprises at least one further ingredient selected from the group consisting of a builder, surfactant, anionic polymer and combinations thereof, and/or **in that** it comprises at least one further enzyme, in particular a protease, amylase, cellulase, pectin-splitting enzyme, hemicellulase, mannanase, tannase, xylanase, xanthanase, β-glucosidase, carrageenase, perhydrolase, oxidase, oxidoreductase, or a lipase, as well as combinations thereof, in particular a combination selected from protease and amylase, protease and lipase, protease and cellulase, protease and mannanase, amylase and lipase, amylase and cellulase, amylase and mannanase, lipase and cellulase, lipase and mannanase, lipase and cellulase, protease and amylase and lipase, protease and amylase and cellulase, protease and amylase and mannanase, amylase and lipase and cellulase, amylase and lipase and mannanase, lipase, cellulase, and mannanase, protease and amylase and lipase and cellulase, protease and amylase and cellulase and mannanase.

7. Method for removing stains, in particular tea stains, from hard surfaces, in particular dishes, comprising one of the following steps
(a) bringing the hard surface into contact with a cleaning solution containing a dishwashing detergent according to one of claims 1 to 6, or
(b) bringing the hard surface into contact with a cleaning solution comprising a hydrogen peroxide source, a bleaching catalyst, and a protease, wherein the protease comprises an amino acid sequence identical to that shown in SEQ ID NO. 3, wherein the bleaching catalyst is selected from the group consisting of complexes of manganese with 1,4,7-trimethyl-1,4,7-triazacyclononane (Me-TACN) or 1,2,4,7-tetramethyl-1,4,7-triazacyclononane (Me/Me-TACN), and wherein the hydrogen peroxide source is sodium percarbonate.

8. Use of a dishwashing detergent according to any one of claims 1 to 6 for removing stains, in particular tea stains, on hard surfaces, or a protease comprising an amino acid sequence identical to that shown in SEQ ID NO. 3, for removing stains, in particular tea stains, from hard surfaces in a cleaning solution, wherein the cleaning solution further comprises a hydrogen peroxide source and a bleaching catalyst, wherein the bleaching catalyst is selected from the group of manganese complexes with 1,4,7-trimethyl-1,4,7-triazacyclononane (Me-TACN) or 1,2,4,7-tetramethyl-1,4,7-triazacyclononane (Me/Me-TACN), and wherein the hydrogen peroxide source is sodium percarbonate.

## Revendications

1. Produit vaisselle comprenant une source de peroxyde d'hydrogène, un catalyseur de blanchiment et une protéase, **caractérisé en ce que**
le catalyseur de blanchiment est choisi dans le groupe des complexes de manganèse avec le 1,4,7-triméthyl-1,4,7-triazacyclononane (Me-TACN) ou le 1,2,4,7-tétraméthyl-1,4,7-triazacyclononane (Me/Me-TACN),
la protéase comprend une séquence d'acides aminés identique à la séquence d'acides aminés indiquée dans SEQ ID NO. 3 et
la source de peroxyde d'hydrogène est le percarbonate de sodium.

2. Produit de lavage de vaisselle selon la revendication 1, **caractérisé en ce qu'**il
la protéase est contenue en une quantité de 1 x 10⁻⁸ à 10 % en poids par rapport à la teneur totale en protéines de la protéase, et/ou
le catalyseur de blanchiment en une quantité de 0,0025 à 1 % en poids, et/ou
la source de peroxyde d'hydrogène dans une quantité de 2 à 30 % en poids.

3. Produit de lavage pour vaisselle selon l'une des revendications 1 à 2, **caractérisé en ce qu'**il comprend en outre un activateur de blanchiment, en particulier du TAED, de préférence en une quantité de 0,1 à 10 % en poids.

4. Produit vaisselle selon l'une des revendications 1 à 3, **caractérisé en ce qu'**il s'agit d'un produit vaisselle pour lave-vaisselle.

5. Produit vaisselle selon l'une des revendications 1 à 4, **caractérisé en ce qu'**il se présente sous forme solide, en particulier sous forme de poudre fluide ou de corps moulé, ou **en ce qu'**il se présente sous forme liquide, gélifiée ou pâteuse.

6. Produit vaisselle selon l'une des revendications 1 à 5, **caractérisé en ce qu'**il comprend au moins un autre ingrédient choisi dans le groupe constitué par un adjuvant, un tensioactif, un polymère anionique et des combinaisons de ceux-ci, et/ou **en ce qu'**il comprend au moins une autre enzyme, en particulier une protéase, amylase, une cellulase, une enzyme clivant la pectine, une hémicellulase, une mannanase, une tannase, une xylanase, xanthane, β-glucosidase, carragénane, perhydrolase, oxydase, oxydoréductase ou une lipase, ainsi que des combinaisons de celles-ci, en particulier une combinaison choisie parmi la protéase et l'amylase, la protéase et la lipase, la protéase et la cellulase, la protéase et la mannanase, l'amylase et la lipase, amylase et cellulase, amylase et mannanase, lipase et cellulase, lipase et mannanase, lipase et cellulase, protéase et amylase et lipase, protéase et amylase et cellulase, protéase et amylase et mannanase, amylase et lipase et cellulase, amylase et lipase et mannanase, lipase, cellulase et mannanase, protéase et amylase et lipase et cellulase, protéase et amylase et cellulase et mannanase.

7. Procédé pour éliminer les salissures, en particulier les taches de thé, sur des surfaces dures, en particulier la vaisselle, comprenant l'une des étapes suivantes
(a) la mise en contact de la surface dure avec un liquide de nettoyage contenant un produit vaisselle selon l'une des revendications 1 à 6, ou
(b) mettre en contact la surface dure avec une solution nettoyante comprenant une source de peroxyde d'hydrogène, un catalyseur de blanchiment et une protéase, la protéase comprenant une séquence d'acides aminés identique à celle indiquée dans SEQ ID NO. 3, le catalyseur de blanchiment étant choisi dans le groupe des complexes de manganèse avec le 1,4,7-triméthyl-1,4,7-triazacyclononane (Me-TACN) ou le 1,2,4,7-tétraméthyl-1,4,7-triazacyclononane (Me/Me-TACN), et dans lequel la source de peroxyde d'hydrogène est le percarbonate de sodium.

8. Utilisation d'un produit vaisselle selon l'une des revendications 1 à 6 pour éliminer les salissures, en particulier les taches de thé, sur des surfaces dures, ou d'une protéase qui comprend une séquence d'acides aminés identique à celle indiquée dans SEQ ID NO. 3, pour éliminer les salissures, en particulier les taches de thé, sur des surfaces dures dans un bain de nettoyage, le bain de nettoyage comprenant en outre une source de peroxyde d'hydrogène et un catalyseur de blanchiment, le catalyseur de blanchiment étant choisi dans le groupe des complexes de manganèse avec le 1,4,7-triméthyl-1,4,7-triazacyclononane (Me-TACN) ou de 1,2,4,7-tétraméthyl-1,4,7-triazacyclononane (Me/Me-TACN), et dans lequel la source de peroxyde d'hydrogène est le percarbonate de sodium.


### SEQUENCE LISTING

<110> Henkel AG & Co. KGaA
<120> Geschirrspülmittel mit Bleichkatalysator und Protease
<130> PT019138 PCT
<150> DE 102011118037.4
   <151> 2011-06-16
<160> 3
<170> PatentIn version 3.5
<210> 1
   <211> 269
   <212> PRT
   <213> Bacillus lentus
<400> 1
   Ala Gln Ser Val Pro Trp Gly Ile Ser Arg Val Gln Ala Pro Ala Ala
   1               5                   10                  15
   His Asn Arg Gly Leu Thr Gly Ser Gly Val Lys Val Ala Val Leu Asp
               20                  25                  30
   Thr Gly Ile Ser Thr His Pro Asp Leu Asn Ile Arg Gly Gly Ala Ser
           35                  40                  45
   Phe Val Pro Gly Glu Pro Ser Thr Gln Asp Gly Asn Gly His Gly Thr
       50                  55                  60
   His Val Ala Gly Thr Ile Ala Ala Leu Asn Asn Ser Ile Gly Val Leu
   65                  70                  75                  80
   Gly Val Ala Pro Ser Ala Glu Leu Tyr Ala Val Lys Val Leu Gly Ala
                   85                  90                  95
   Ser Gly Ser Gly Ser Val Ser Ser Ile Ala Gln Gly Leu Glu Trp Ala
               100                 105                 110
   Gly Asn Asn Gly Met His Val Ala Asn Leu Ser Leu Gly Ser Pro Ser
           115                 120                 125
   Pro Ser Ala Thr Leu Glu Gln Ala Val Asn Ser Ala Thr Ser Arg Gly
       130                 135                 140
   Val Leu Val Val Ala Ala Ser Gly Asn Ser Gly Ala Gly Ser Ile Ser
   145                 150                 155                 160
   Tyr Pro Ala Arg Tyr Ala Asn Ala Met Ala Val Gly Ala Thr Asp Gln
                   165                 170                 175
   Asn Asn Asn Arg Ala Ser Phe Ser Gln Tyr Gly Ala Gly Leu Asp Ile
               180                 185                 190
   Val Ala Pro Gly Val Asn Val Gln Ser Thr Tyr Pro Gly Ser Thr Tyr
           195                 200                 205
   Ala Ser Leu Asn Gly Thr Ser Met Ala Thr Pro His Val Ala Gly Ala
       210                 215                 220
   Ala Ala Leu Val Lys Gln Lys Asn Pro Ser Trp Ser Asn Val Gln Ile
   225                 230                 235                 240
   Arg Asn His Leu Lys Asn Thr Ala Thr Ser Leu Gly Ser Thr Asn Leu
                   245                 250                 255
   Tyr Gly Ser Gly Leu Val Asn Ala Glu Ala Ala Thr Arg
               260                 265
<210> 2
   <211> 269
   <212> PRT
   <213> Bacillus sp.
<400> 2
   Gln Gln Thr Val Pro Trp Gly Ile Thr Arg Val Gln Ala Pro Thr Val
   1               5                   10                  15
   His Asn Arg Gly Val Thr Gly Ser Gly Val Lys Val Ala Ile Leu Asp
               20                  25                  30
   Thr Gly Ile Ala Gln His Ser Asp Leu Thr Ile Arg Gly Gly Ala Ser
           35                  40                  45
   Phe Val Pro Gly Glu Ser Thr Thr Ala Asp Leu Asn Gly His Gly Thr
       50                  55                  60
   His Val Ala Gly Thr Val Ala Ala Leu Asn Asn Ser Ile Gly Val Ile
   65                  70                  75                  80
   Gly Val Ala Pro Ser Ala Asp Leu Tyr Ala Val Lys Val Leu Gly Ala
                   85                  90                  95
   Asn Gly Arg Gly Ser Val Ser Gly Ile Ala Gln Gly Leu Glu Trp Ala
               100                 105                 110
   Ala Thr Asn Asn Met His Ile Ala Asn Met Ser Leu Gly Ser Asp Ala
               115                 120                 125
   Pro Ser Thr Thr Leu Glu Arg Ala Val Asn Tyr Ala Thr Ser Arg Gly
       130                 135                 140
   Val Leu Val Ile Ala Ala Thr Gly Asn Asn Gly Thr Gly Ser Ile Gly
   145                 150                 155                 160
   Tyr Pro Ala Arg Tyr Ala Asn Ala Met Ala Val Gly Ala Thr Asp Gln
                   165                 170                 175
   Asn Asn Arg Arg Ala Ser Phe Ser Gln Tyr Gly Thr Gly Ile Asp Ile
               180                 185                 190
   Val Ala Pro Gly Val Gly Ile Gln Ser Thr Tyr Leu Asn Asn Ser Tyr
           195                 200                 205
   Ala Ser Met Pro Gly Thr Ser Met Ala Thr Pro His Val Ala Gly Val
       210                 215                 220
   Ala Ala Leu Val Lys Gln Lys Asn Pro Ser Trp Asn Ala Thr Gln Ile
   225                 230                 235                 240
   Arg Asn His Leu Lys Asn Thr Ala Thr Asn Leu Gly Asn Ser Ser Gln
                   245                 250                 255
   Phe Gly Ser Gly Leu Val Asn Ala Asp Ala Ala Thr Arg
               260                 265
<210> 3
   <211> 269
   <212> PRT
   <213> Bacillus sp.
<400> 3
   Ala Gln Ser Val Pro Trp Gly Ile Arg Arg Val Gln Ala Pro Thr Ala
   1               5                   10                  15
   His Asn Arg Gly Leu Thr Gly Ser Gly Val Lys Val Ala Val Leu Asp
               20                  25                  30
   Thr Gly Ile Ser Thr His Pro Asp Leu Asn Ile Arg Gly Gly Ala Ser
           35                  40                  45
   Phe Val Pro Gly Glu Pro Ser Thr Gln Asp Gly Asn Gly His Gly Thr
       50                  55                  60
   His Ala Ala Gly Thr Ile Ala Ala Leu Asn Asn Ser Ile Gly Val Leu
   65                  70                  75                  80
   Gly Val Ala Pro Ser Ala Glu Leu Tyr Ala Val Lys Val Leu Gly Ala
                   85                  90                  95
   Ser Gly Ser Gly Ser Val Ser Ser Ile Ala Gln Gly Leu Glu Trp Ala
               100                 105                 110
   Gly Asn Asn Gly Met His Val Ala Asn Leu Ser Leu Gly Ser Pro Ser
           115                 120                 125
   Pro Ser Ala Thr Leu Glu Gln Ala Val Asn Ser Ala Thr Ser Arg Gly
       130                 135                 140
   Val Leu Val Val Ala Ala Ser Gly Asn Ser Gly Ala Gly Ser Ile Ser
   145                 150                 155                 160
   Tyr Pro Ala Arg Tyr Ala Asn Ala Met Ala Val Gly Ala Thr Asp Gln
                   165                 170                 175
   Asn Asn Asn Arg Ala Ser Phe Ser Gln Tyr Gly Ala Gly Leu Asp Ile
               180                 185                 190
   Val Ala Pro Gly Val Asn Val Gln Ser Thr Tyr Pro Gly Ser Thr Tyr
           195                 200                 205
   Ala Ser Leu Asp Gly Thr Ser Met Ala Thr Pro His Val Ala Gly Ala
       210                 215                 220
   Ala Ala Leu Val Lys Gln Lys Asn Pro Ser Trp Ser Asn Val Arg Ile
   225                 230                 235                 240
   Arg Asn His Leu Lys Asn Thr Ala Thr Ser Leu Gly Ser Thr Asn Leu
                   245                 250                 255
   Tyr Gly Ser Gly Leu Val Asn Ala Glu Ala Ala Thr Arg
               260                 265